# EUROPEAN PATENT APPLICATION

(11) **EP 3 960 098 A1**
(43) Date of publication of application: **02.03.2022**
(21) Application number: 20193342.1
(22) Date of filing: 28.08.2020
(51) Int. Cl.: A61B 17/22

(54) **APPARATUS FOR AN ENDOUROLOGICAL PROCEDURE**

(71) Applicant: Advanced MedTech Corporate Pte. Ltd., Singapore 608526 (SG)
(72) Inventor: TAN, Wui Siew, 608526 Singapore (SG)
(74) Representative: HGF

(57) **Abstract**

The present application provides an access sheath (2) for use in an endourological procedure. The access sheath (2) is configured for use with a surgical tool (4) having a suction channel (8) for extraction of fluid from a treatment site (5) during use. The access sheath (2) is positionable within a patient's urinary system (3) during use to provide access to the treatment site (5) for the surgical tool (4). The access sheath (2) comprises an access channel (6) extending to a distal end (10) of the access sheath (2). The access channel (6) is configured to receive the surgical tool (4) such that a distal end (11) of the surgical tool (4) passes through the access channel (6) and is positioned at the treatment site (5) during use. The access sheath (2) also includes an irrigation fluid channel (7) configured to convey irrigation fluid to the treatment site (7) during use such that irrigation fluid is provided to the treatment site (5) via the irrigation fluid channel (7) of the access sheath (2) and is extracted via the suction channel (8) of the surgical tool (6). The present application also provides apparatus (1) for use in an endourological procedure that includes a surgical tool (4) and an access sheath (2), and an access sheath adapter (35) for use with the access sheath (2).

## Description

This disclosure relates to apparatus for an endourological procedure, for example minimally invasive kidney stone treatment. In particular, this disclosure relates to an access sheath, an access sheath adaptor, and apparatus for use in an endourological procedure.

### BACKGROUND

During endourological procedures (e.g. ureteroscopy, percutaneous nephrolithotomy (PCNL)) surgical tools may be passed through a narrow opening into the body multiple times to effect the surgical treatment within the body. An access sheath is used to protect the conduit through which these tools are passed, for example a patient's urethra, bladder, and ureter. In particular during retrograde intrarenal surgery (RIRS), an access sheath is passed through the patient's urethra, through the bladder, and up the ureter to provide access to the patient's kidney for tools such as a ureteroscope, basket, etc.. Alternatively, during PCNL for example, the access sheath extends from outside of the patient through the skin, muscle, and kidney tissue (renal capsule) into the renal pelvis of the kidney to access the kidney calyces, ureter, and bladder. The access sheath creates a channel through which tubular surgical tools can be passed to reach the desired location within the patient's urinary system, for example the kidney. The access sheath provides protection of the surrounding tissue (e.g. the ureter for ureteroscopy, or the layer of muscle, connective, and kidney tissue for PCNL). The access sheath can also reduce the risk of pressure-induced damage to the kidney by providing a path for outflow of fluid from the kidney. The access sheath can also prevent over-extension of the ureter.

For RIRS lithotripsy using a medical laser, a ureteroscope is first passed through the access sheath to visualize the kidney stone location, and a medical laser (e.g., a laser fibre or lightguide) is then passed through a working channel of the ureteroscope to deliver the laser pulses in proximity to the kidney stone. The ureteroscope includes a camera and light source providing a video feed to an operator.

Typically, during RIRS lithotripsy procedure irrigation fluid is passed through an irrigation channel of the ureteroscope, and passive fluid outflow occurs through a gap between the ureteroscope and the inner surface of the access sheath. Optionally, suction can be applied to the access sheath to provide suction through the gap between the ureteroscope and the access sheath to extract fluid and kidney stone fragments from the kidney. Such passive or active outflow via the access sheath can occur with or without presence of other tools positioned in the access sheath. During a procedure the operator adjusts the flow of the irrigation fluid into the irrigation channel of the ureteroscope through a gravity driven method of raising or lowering a bag of saline that drains into the ureteroscope.

### BRIEF SUMMARY OF THE DISCLOSURE

In accordance with the present disclosure there is provided an access sheath for use in an endourological procedure, wherein the access sheath is configured for use with a surgical tool having a suction channel. The access sheath is positionable within a patient's urinary system during use to provide access to a treatment site for the surgical tool. The access sheath comprises an access channel extending to a distal end of the access sheath. The access channel is configured to receive the surgical tool such that a distal end of the surgical tool passes through the access channel and is positioned at the treatment site during use. The access sheath also comprises an irrigation fluid channel configured to convey irrigation fluid to the treatment site during use. Accordingly, during use irrigation fluid is provided to the treatment site via the irrigation fluid channel of the access sheath and fluid exits via the channel of the surgical tool.

The access sheath may be configured for use in ureteroscopy, where the access sheath is passed through a patient's urethra and bladder and into the ureter. As described further below, during ureteroscopy the access sheath may extend into the renal pelvis or kidney. For ureteroscopy the surgical tool may be a ureteroscope having a suction channel, and the access channel of the access sheath is configured to receive the ureteroscope. The ureteroscopy procedure may include retrograde intrarenal surgery (RIRS).

Alternatively, the access sheath may be configured for use in percutaneous nephrolithotomy (PCNL), for example mini PCNL or micro PCNL, where the access sheath is passed through a surgical opening formed in the patient's body and into the kidney to provide access for the surgical tool.

The apparatus, in particular the access sheath, provides irrigation fluid and suction during the endourological procedure in an opposite direction to what is currently known. In particular, providing irrigation fluid via an irrigation fluid channel in the access sheath, and suction via a suction channel in a surgical tool, is a reverse of currently commercially available endourology apparatus. This reverse flow direction provides several advantages. In particular, during an endourological procedure using an endoscope or ureteroscope the reverse flow direction keeps debris away from any camera in the surgical tool. The reverse flow direction also tends to keep the kidney stone closer to the endoscope or ureteroscope, rather than pushing it away, which is advantageous for lithotripsy procedures and for procedures to inspect kidney stones. The reverse flow direction additionally ensures that debris is primarily entrained in irrigation fluid flowing towards the suction channel because the suction channel is aligned with the endoscope or ureteroscope in the access channel, and so is aligned with any objects, such as kidney stones, that are being inspected, ablated, and/or extracted. Therefore, debris is not circulated around the kidney by inflowing irrigation fluid and can be more easily extracted.

Importantly, in this reverse flow configuration, larger fragments can be extracted through the suction channel of the surgical tool than through the small and unpredictable space between the surgical tool and the access sheath as is typical. Typically, the space between a ureteroscope and access sheath inner diameter is at maximum 0.3-1 mm wide, and the positions and movement of surgical tools in the access sheath means this can be reduced or obstructed along the length of the access sheath, reducing the space to as little as 0.15mm - 0.5 mm. Providing the suction channel in the ureteroscope and the irrigation fluid channel in the access sheath means that the suction channel of the surgical tool can be a uniform cylindrical conduit with a larger diameter, for example about 1 mm to about 2 mm or more, and can be operated without additional tools disposed in the suction channel.

In examples, the access sheath may comprise a plurality of irrigation fluid channels. Each of the plurality of irrigation fluid channels may be configured to convey irrigation fluid to the treatment site during use. Providing multiple irrigation fluid channels provides improved fluid flow at the treatment site.

In examples, at least two of the plurality of irrigation fluid channels have different shaped or size openings at the distal end of the access sheath, and/or different diameters or sizes along their length. Accordingly, irrigation fluid provided via these channels will be output to the treatment area at different flow rates, fluid velocities, and pressures. Additionally or alternatively, at least two of the plurality of irrigation fluid channels have different internal surface properties providing different fluid resistance to irrigation fluid flowing therethrough, so that irrigation fluid provided via these channels will be output to the treatment area at different flow rates and pressures. Accordingly, one or more irrigation fluid channels can be selectively operated to provide variation, or control, over the flow field of irrigation fluid at the treatment site.

In examples, the access channel and the or each irrigation fluid channel may be movable relative to each other within the access sheath such that the respective positions of the access channel and the or each irrigation channel can be altered to vary the fluid flow field at the treatment site. Accordingly, an operator can vary, or control, the fluid flow at the treatment site to improve visibility or move objects, such as kidney stones, that are lodged. For example, a kidney stone may be lodged in the lower pole of the kidney or within pockets where the kidney medullary pyramids connect to the renal calyces. In some examples, the operator can vary, or control, the speed and/or direction of the irrigation fluid flow at the treatment site to dislodge such kidney stones.

In some examples the access channel and the or each irrigation fluid channel are separate lumens in the access sheath. The access sheath may comprise an elongate tubular body having a first lumen defining the access channel and one or more second lumens defining the or each irrigation fluid channel. Such an access sheath can be produced by an extrusion process.

In examples, the access sheath may comprise a lumen extending to the distal end of the access sheath, the lumen having a non-circular cross-section configured to constrain the position of the surgical tool during use. In this example, the or each irrigation fluid channel is defined within the lumen in an area surrounding the surgical tool during use. The lumen may comprise one or more sidewall protrusions defining the non-circular cross-section of the lumen. The one or more sidewall protrusions may contact the surgical tool during use to constrain the position of the surgical tool within the lumen. The one or more sidewall protrusions define spaces therebetween where the surgical tool is too large to enter, and so these spaces may form the one or more irrigation fluid channels. The sidewall protrusion(s) may abut the surgical tool and seal against it such that the irrigation fluid channel(s) are separate within the spaces between the sidewall protrusions. A membrane may be attached to the or each sidewall protrusion(s) and the one or more irrigation fluid channels may be defined between the membrane and the access sheath. The membrane provides separate irrigation fluid channels so the irrigation fluid channels are separate even when no surgical tool is present in the access channel.

In examples, the access sheath may comprise a rotatable insert received in a distal end of the access channel and configured to rotationally couple to the surgical tool during use. The rotatable insert may comprise one or more blocking members that are arranged to block the or each irrigation fluid channel on rotation of the surgical tool and rotatable insert.

In examples, the access sheath may further comprise a coupling mechanism configured to couple the access sheath to the surgical tool during use.

According to a further aspect of the present disclosure there is provided an access sheath for use with a surgical tool. The access sheath comprises an elongate body adapted to be positioned within a patient's urinary system during use, a first lumen extending to a distal end of the elongate body, the first lumen being configured to receive the surgical tool such that a distal end of the surgical tool passes through the first lumen and is positioned at the treatment site during use. The access sheath also comprises a coupling mechanism configured to couple the access sheath to the surgical tool as the surgical tool is passed into the lumen during use.

Coupling the access sheath to the surgical tool during use allows for the distal end of the access sheath to be moved with the surgical tool towards the kidney, or even into the kidney. In some examples, the coupling mechanism is disposed at or near the distal end of the access sheath. This ensures that the irrigation fluid channel(s) are aligned with, or close to, the distal end of the surgical tool during use so that the irrigation fluid is provided at the treatment site to improve operation of the surgical tool.

Such an example is advantageous for ureteroscopy procedures, where an access sheath is typically positioned in the patient's urethra, bladder and partly along the ureter, or at most projecting slightly out of the ureter into the renal pelvis. The coupling mechanism provides for moving the distal end of the access sheath into, or further into, the renal pelvis of the kidney during use, as the ureteroscope is passed through the access sheath. A distal end portion of the access sheath may be flexible and/or extendible such that the distal end portion access sheath can be moved into the renal pelvis and closer to the treatment site. Accordingly, the distal end of the access sheath can be positioned at the treatment site and articulated together with the ureteroscope.

In some examples the coupling mechanism is configured to decouple the access sheath from the surgical tool on retraction of the surgical tool away from the treatment site, out of the body. In some examples, the coupling mechanism may be configured to selectively decouple the access sheath and the surgical tool.

In examples, the coupling mechanism may be configured to couple the access sheath to the surgical tool as the surgical tool is passed through the access channel towards the treatment site during use, and decouple the access sheath from the surgical tool as the surgical tool is retracted away from the treatment site during or after use. In some examples, the coupling mechanism is operable to selectively couple and decouple the access sheath from the surgical tool.

In examples, the coupling mechanism may comprise a resiliently deformable annular membrane disposed in the access channel. The resiliently deformable annular membrane is configured to permit the distal end of the surgical tool to pass through the annular membrane and grip a side of the surgical tool as the surgical tool is passed through the access channel during use. Once the resiliently deformable annular membrane has gripped the surgical tool, movement of the surgical tool towards the kidney will also move the access sheath, or a flexible/extendible distal end portion of the access sheath, in the same direction. Similarly, movement of the surgical tool away from the kidney will also move the access sheath or a flexible/extendible distal end portion of the access sheath. To decouple the access sheath from the surgical tool an operator can hold the proximal end of the access sheath and move the surgical tool relative to the access sheath so that the resiliently deformable annular membrane can release or move over the surgical tool.

In some examples the coupling mechanism comprises a coupling member extending into the access channel. The coupling member may be configured to engage the surgical tool to couple the access sheath to the surgical tool. In examples, the coupling member is configured to engage a corresponding coupling feature of the surgical tool. The coupling member may comprise one or more protrusions at or near the distal end of the access sheath, or an annular ring at or near the distal end of the access sheath. The coupling member(s) may be abutted by the surgical tool as the surgical tool is passed along the access channel during use. Subsequent movement of the surgical tool towards the kidney pushes the access sheath to move it along with the surgical tool. When the surgical tool is retracted away from the kidney the coupling member disengages allowing the surgical tool to be retracted without the access sheath.

In similar examples, the coupling mechanism comprises one or more protrusions in the access sheath that are configured to engage one or more coupling grooves in the surgical tool, the one or more coupling grooves being spaced from a distal end of the surgical tool. In these examples, the distal end of the surgical tool is configured to pass by the one or more protrusions in a first rotational position relative to the access sheath, and rotation of the surgical tool causes the one or more protrusions to be received in the one or more grooves, thereby coupling the surgical tool to the access sheath. The surgical tool can subsequently be decoupled from the access sheath by rotation of the surgical tool to move the one or more protrusions out of the one or more grooves. In some examples, the one or more coupling grooves in the surgical tool may be L shaped, such that the one or more protrusions can pass along a straight portion of the one or more coupling grooves, and then rotation of the surgical tool moves the one or more protrusions into the leg(s) of the one or more L-shaped coupling grooves to provide axial coupling of the surgical tool to the access sheath.

In some examples, a distal end portion of the access sheath that is coupled to the surgical tool during use is flexible to permit articulation of the surgical tool.

In some examples, the distal end portion of the access sheath is extendible, for example resiliently extendible, such that the distal end portion can be extended relative to the remainder of the access sheath. During use, the extensibility of the distal end portion permits movement of the surgical tool and the distal end of the access sheath towards the treatment site without moving the remainder of the access sheath.

According to a further aspect of the present disclosure there is provided an access sheath adapter for use with the access sheath described above during an endourological procedure. The access sheath adapter comprises a connection portion for connecting to a proximal end of the access sheath, an opening providing access for the surgical tool, and an irrigation fluid supply connector for connecting the or each irrigation fluid channel to a source of irrigation fluid. Accordingly, irrigation fluid is provided to the or each irrigation fluid channel via the access sheath adapter, such as via the irrigation fluid supply connector.

In examples where the access sheath comprises a plurality of irrigation fluid channels, the irrigation fluid supply connector may comprise independent irrigation fluid connections between the source of irrigation fluid and each of the plurality of irrigation fluid channels. Accordingly, irrigation fluid can be selectively provided to each of the irrigation fluid channels independently of each other. Each of the irrigation fluid connections may be independently openable and closable. For example, the access sheath adapter may further comprise a plurality of valves arranged such that each of the plurality of irrigation fluid channels can be independently opened or closed. In other examples, an upstream irrigation fluid system may selectively provide irrigation fluid to each of the irrigation fluid channels.

Selectively supplying irrigation fluid to the plurality of irrigation fluid channels allows for varying and controlling a fluid flow field at the treatment site by, for example, turning on or off some of the plurality of irrigation fluid channels, or changing a pressure or flow rate of irrigation fluid through some of the plurality of irrigation fluid channels. For example, if an operator's camera view is obscured by debris then the fluid flow field may be varied by changing which irrigation fluid channels are open and closed, which may move the debris away from the camera lens. Also, the fluid flow field may be controlled to help dislodge a trapped piece of material (e.g. kidney stone) by directing flow in an appropriate direction at the treatment site.

The access sheath adapter may comprise an internal sealed chamber divided into two or more sub-chambers, wherein each sub-chamber is in fluid communication with a different irrigation fluid channel of the access sheath. Each sub-chamber may have an irrigation fluid inlet connected to a source of irrigation fluid, and supply of irrigation fluid to each sub-chamber may be controllable to determine which irrigation fluid channel(s) are provided with irrigation fluid. Each sub-chamber may be in fluid communication with more than one irrigation fluid channel.

In some examples, the access sheath adapter may comprise a separate surgical tool channel for the surgical tool to access the access channel in the access sheath. The surgical tool channel may extend through one or more of the sub-chambers to provide a sealed path for the surgical tool to access the access channel of the access sheath.

According to a further aspect of the present disclosure there is provided apparatus for use in an endourological procedure. The apparatus comprises a surgical tool comprising a suction channel for extraction of fluid from a treatment site during use, and an access sheath positionable within a patient's urinary system during use to provide access to the treatment site for the surgical tool. The access sheath comprises an access channel extending to a distal end of the access sheath. The access channel is configured to receive the surgical tool such that a distal end of the surgical tool passes through the access channel and is positioned at the treatment site during use. The access sheath further comprises an irrigation fluid channel configured to convey irrigation fluid to the treatment site during use such that irrigation fluid is provided to the treatment site via the irrigation fluid channel of the access sheath and is extracted via the suction channel of the surgical tool.

Providing irrigation fluid through the access sheath and suction through the surgical tool is an opposite flow direction to what is currently known. In particular, providing irrigation fluid via an irrigation fluid channel in the access sheath, and suction via a suction channel in a surgical tool, is a reverse of currently commercially available endourological apparatus. This reverse flow direction provides several advantages. In particular, during an endourological procedure the reverse flow direction keeps debris away from any camera in the surgical tool (e.g., ureteroscope). The reverse flow direction also tends to keep the kidney stone closer to the surgical tool, rather than pushing it away, which is advantageous for lithotripsy procedures and for procedures to inspect kidney stones. The reverse flow direction additionally ensures that debris is primarily entrained in irrigation fluid flowing towards the suction channel because the suction channel is aligned with the surgical tool in the access channel, and so is aligned with any objects, such as kidney stones, that are being inspected, ablated, or extracted. Therefore, debris is not circulated around the kidney by inflowing irrigation fluid and can be more easily extracted. Furthermore, the reverse flow direction allows for removal of larger pieces of debris because the suction channel in the surgical tool is inherently larger and has a more consistent diameter compared to an outflow channel defined in the space between the access sheath and surgical tool.

As described below, the access sheath of the apparatus may comprise any of the features described above with respect to the access sheath.

In examples, the access sheath may comprise a plurality of irrigation fluid channels. Each of the plurality of irrigation fluid channels may be configured to convey irrigation fluid to the treatment site during use. Providing multiple irrigation fluid channels provides improved fluid flow at the treatment site.

In examples, at least two of the plurality of irrigation fluid channels have different shaped or size openings at the distal end of the access sheath, and/or different diameters or sizes along their length. Accordingly, irrigation fluid provided via these channels will be output to the treatment area at different flow rates, fluid velocities, and pressures. Additionally or alternatively, at least two of the plurality of irrigation fluid channels have different internal surface properties providing different fluid resistance to irrigation fluid flowing therethrough, so that irrigation fluid provided via these channels will be output to the treatment area at different flow rates and pressures. Accordingly, one or more irrigation fluid channels can be selectively operated to provide variation, or control, over the flow field of irrigation fluid at the treatment site.

In examples, the access channel and the or each irrigation fluid channel may be movable relative to each other within the access sheath such that the respective positions of the access channel and the or each irrigation channel can be altered to vary the fluid flow field at the treatment site. Accordingly, an operator can vary, or control, the fluid flow at the treatment site to improve visibility or move objects, such as kidney stones, that are lodged. For example, a kidney stone may be lodged within pockets where the kidney medullar pyramids connect to the renal calyces. In examples, the operator may vary, or control, the speed and/or direction of the irrigation fluid at the treatment site, for example to dislodge trapped particles or to move them into favourable locations.

In some examples the access channel and the or each irrigation fluid channel are separate lumens in the access sheath. The access sheath may comprise an elongate tubular body having a first lumen defining the access channel and one or more second lumens defining the or each irrigation fluid channel. Such an access sheath can be produced by an extrusion process.

In examples, the access sheath may comprise a lumen extending to the distal end of the access sheath, the lumen having a non-circular cross-section configured to constrain the position of the surgical tool during use. In this example, the or each irrigation fluid channel is defined within the lumen in an area surrounding the surgical tool during use. The lumen may comprise one or more sidewall protrusions defining the non-circular cross-section of the lumen. The one or more sidewall protrusions may contact the surgical tool during use to constrain the position of the surgical tool within the lumen. The one or more sidewall protrusions define spaces therebetween where the surgical tool is too large to enter, and so these spaces may form the one or more irrigation fluid channels. The sidewall protrusion(s) may about the surgical tool and seal against it such that the irrigation fluid channel(s) are separate within the spaces between the sidewall protrusions. In some examples, a membrane may be attached to the or each sidewall protrusion(s) and the one or more irrigation fluid channels may be defined between the membrane and the access sheath. The membrane provides separate irrigation fluid channels so the irrigation fluid channels are separate even when no surgical tool is present in the access channel.

In examples, the access sheath may comprise a rotatable insert received in a distal end of the access channel and configured to rotationally couple to the surgical tool during use. The rotatable insert may comprise one or more blocking members that are arranged to block the or each irrigation fluid channel on rotation of the surgical tool and rotatable insert.

In examples, the access sheath may further comprise a coupling mechanism configured to couple the access sheath to the surgical tool during use.

The access sheath may further comprise a coupling mechanism disposed in the access channel and configured to couple the access sheath to the surgical tool as the surgical tool is passed into the lumen during use.

Coupling the access sheath to the surgical tool during use allows for the access sheath to be moved with the surgical tool towards and into the kidney. In some examples, the coupling mechanism is disposed at or near the distal end of the access sheath. This ensures that the irrigation fluid channel(s) are aligned with, or close to, the distal end of the surgical tool during use so that the irrigation fluid is provided at the treatment site to improve operation of the surgical tool.

Such an example is particularly advantageous for ureteroscopy procedures, where an access sheath is typically positioned in the patient's urethra, bladder and partly along the ureter, or at most projecting slightly out of the ureter. The coupling mechanism provides for moving the end of the access sheath into, or further into, the renal pelvis of the kidney during use, as the ureteroscope is passed through the access sheath. A distal end portion of the access sheath may be flexible and/or extendible such that the distal end portion access sheath can be moved into the renal pelvis and closer to the treatment site. Accordingly, the distal end of the access sheath can be positioned at the treatment site and articulated together with the ureteroscope.

In some examples the coupling mechanism is configured to decouple the access sheath from the surgical tool on retraction of the surgical tool away from the treatment site, out of the body. In some examples, the coupling mechanism may be configured to selectively decouple the access sheath and the surgical tool.

In examples, the coupling mechanism may be configured to couple the access sheath to the surgical tool as the surgical tool is passed through the access channel towards the treatment site during use, and decouple the access sheath from the surgical tool as the surgical tool is retracted away from the treatment site during or after use. In some examples, the coupling mechanism is operable to selectively couple and decouple the access sheath from the surgical tool.

In examples, the coupling mechanism may comprise a resiliently deformable annular membrane disposed in the access channel. The resiliently deformable annular membrane is configured to permit the distal end of the surgical tool to pass through the annular membrane and grip a side of the surgical tool as the surgical tool is passed through the access channel during use. Once the resiliently deformable annular membrane has gripped the surgical tool, movement of the surgical tool towards the kidney will also move the access sheath, or a flexible/extendible distal end portion of the access sheath, in the same direction. Similarly, movement of the surgical tool away from the kidney will also move the access sheath or a flexible/extendible distal end portion of the access sheath. To decouple the access sheath from the surgical tool an operator can hold the proximal end of the access sheath and move the surgical tool relative to the access sheath so that the resiliently deformable annular membrane can release or move over the surgical tool.

In some examples the coupling mechanism comprises a coupling member extending into the access channel. The coupling member may be configured to engage the surgical tool to couple the access sheath to the surgical tool. In examples, the coupling member is configured to engage a corresponding coupling feature of the surgical tool. The coupling member may comprise one or more protrusions at or near the distal end of the access sheath, or an annular ring at or near the distal end of the access sheath. The coupling member(s) may be abutted by the surgical tool as the surgical tool is passed along the access channel during use. Subsequent movement of the surgical tool towards the kidney pushes the access sheath to move it along with the surgical tool. When the surgical tool is retracted away from the kidney the coupling member disengages allowing the surgical tool to be retracted without the access sheath.

In similar examples, the coupling mechanism comprises one or more protrusions in the access sheath that are configured to engage one or more coupling grooves in the surgical tool, the one or more coupling grooves being spaced from a distal end of the surgical tool. In these examples, the distal end of the surgical tool is configured to pass by the one or more protrusions in a first rotational position relative to the access sheath, and rotation of the surgical tool causes the one or more protrusions to be received in the one or more grooves, thereby coupling the surgical tool to the access sheath. The surgical tool can subsequently be decoupled from the access sheath by rotation of the surgical tool to move the one or more protrusions out of the one or more grooves. In some examples, the one or more coupling grooves in the surgical tool may be L shaped, such that the one or more protrusions can pass along a straight portion of the one or more coupling grooves, and then rotation of the surgical tool moves the one or more protrusions into the leg(s) of the one or more L-shaped coupling grooves to provide axial coupling of the surgical tool to the access sheath.

In some examples, a distal end portion of the access sheath that is coupled to the surgical tool during use is flexible to permit articulation of the surgical tool.

In some examples, the distal end portion of the access sheath is extendible, for example resiliently extendible, such that the distal end portion can be extended relative to the remainder of the access sheath. During use, the extensibility of the distal end portion permits movement of the surgical tool and the distal end of the access sheath towards the treatment site without moving the remainder of the access sheath.

The apparatus may also include an access sheath adapter for use with the access sheath and surgical tool. The access sheath adapter may comprise a connection portion for connecting to a proximal end of the access sheath, an opening providing access for the surgical tool, and an irrigation fluid supply connector for connecting the or each irrigation fluid channel to a source of irrigation fluid. Accordingly, irrigation fluid is provided to the or each irrigation fluid channels via the access sheath adapter, in particular via the irrigation fluid supply connector. In some examples, the opening for the surgical tool is a water-tight seal.

In examples where the access sheath comprises a plurality of irrigation fluid channels, the irrigation fluid supply connector may comprise independent irrigation fluid connections between the source of irrigation fluid and each of the plurality of irrigation fluid channels. Accordingly, irrigation fluid can be selectively provided to each of the irrigation fluid channels independently of each other. Each of the irrigation fluid connections may be independently openable and closable. For example, the access sheath adapter may further comprise a plurality of valves arranged such that each of the plurality of irrigation fluid channels can be independently opened or closed. In other examples, an upstream irrigation fluid system may selectively provide irrigation fluid to each of the irrigation fluid channels.

Selectively supplying irrigation fluid to the plurality of irrigation fluid channels allows for varying and controlling a fluid flow field at the treatment site by, for example, turning on or off some of the plurality of irrigation fluid channels, or changing a pressure or flow rate of irrigation fluid through some of the plurality of irrigation fluid channels. For example, if an operator's camera view is obscured by debris then the fluid flow field may be varied by changing which irrigation fluid channels are open and closed, which may move the debris away from the camera lens. Also, the fluid flow field may be controlled to help dislodge a trapped piece of material (e.g. kidney stone) by directing flow in an appropriate direction at the treatment site.

In some examples, the access sheath adapter comprises an internal sealed chamber divided into two or more sub-chambers, wherein each sub-chamber is in fluid communication with a different irrigation fluid channel of the access sheath. Each sub-chamber may have an irrigation fluid inlet connected to a source of irrigation fluid, and supply of irrigation fluid to each sub-chamber may be controllable to determine which irrigation fluid channel(s) are provided with irrigation fluid. Each sub-chamber may be in fluid communication with more than one irrigation fluid channel.

In some examples, the access sheath adapter may comprise a separate surgical tool channel for the surgical tool to access the access channel in the access sheath. The surgical tool channel may extend through one or more of the sub-chambers to provide a sealed path for the surgical tool to access the access channel of the access sheath.

The apparatus may further comprise an irrigation fluid system configured to supply irrigation fluid to the or each irrigation fluid channel. The irrigation fluid system may be a gravity-fed system or it may comprise a pump for providing irrigation fluid to the apparatus, in particular the or each irrigation fluid channel. In examples, a pump can provide a regulated flow of irrigation fluid to the apparatus.

In examples where the access sheath comprises a plurality of irrigation fluid channels, the irrigation fluid system may be configured to selectively provide irrigation fluid to each of the plurality of irrigation fluid channels independently of each other.

In examples the apparatus may further comprise a suction system arranged to generate suction in the suction channel of the surgical tool. The suction system may comprise a suction pump, for example a vacuum pump, or a venturi arrangement for generating suction from a fluid flow (e.g., compressed air).

The apparatus may further comprise a fluid control system configured to control the irrigation fluid system and the suction system. In some examples, the fluid control system may be configured to balance the flow rate of fluid towards, and away from, the treatment site. As described below, the flow rate can be balanced by detecting and controlling a volumetric flow rate, and/or a pressure of the irrigation fluid system and suction system.

According to a further aspect of the present disclosure, there is provided a fluid control system for controlling a fluid flow during an endourological procedure, wherein during the endourological procedure an access sheath is positioned in a patient's urinary system for providing access for a surgical tool. The access sheath has an irrigation fluid channel and the surgical tool has a suction channel. The fluid control system comprises an irrigation fluid system configured to supply irrigation fluid to the irrigation fluid channel of the access sheath such that irrigation fluid is conveyed to a treatment site. The fluid control system also comprises a suction system configured to generate a suction pressure in the suction channel of the surgical tool to extract fluid from the treatment site. The fluid control system is adapted to control the irrigation fluid system and the suction system. The fluid control system may control the irrigation fluid system and the suction system in dependence on each other. That is, the suction system is controlled according to operation of the irrigation fluid system, or vice versa, or both are controlled according to a target fluid flow rate for the endourological procedure. As described further below, the fluid control system may be configured to control the irrigation fluid system and the suction system and in dependence of pressure, temperature, or flow rate information provided by one or more sensors.

In some examples, the fluid control system is configured to control the irrigation fluid system and the suction system. In some examples, the fluid control system may be configured to balance the flow rate of fluid towards, and away from, the treatment site.

In various examples, the fluid flow system, irrigation fluid system, and/or the suction system may be configured to receive an operator input to vary one or more of: an irrigation fluid flow rate, an irrigation fluid pressure, or a suction pressure. The fluid flow system may be configured to control the irrigation fluid system and the suction system according to the operator input and to balance the flow rate of fluid towards, and away from, the treatment site.

In examples where irrigation fluid is selectively provided to a plurality of irrigation fluid channels, then in response to changing a configuration of irrigation fluid channels (e.g., opening, closing, or changing a pressure or flow rate in one or more irrigation fluid channels) the fluid control system is configured to control the suction system to adjust the suction pressure in the suction channel accordingly.

The apparatus for an endourological procedure and/or the fluid control system may further comprise one or more sensors arranged to detect a fluid property of the irrigation fluid flowing towards the treatment site, and/or fluid being extracted through the suction channel. For examples, the apparatus may further comprise a temperature or pressure sensor disposed at a distal end of the access sheath or surgical tool to detect a fluid temperature or pressure at the treatment site. Additionally or alternatively, the apparatus may include a temperature and/or pressure sensor disposed in the irrigation fluid flow path or suction path at the proximal end of the apparatus (e.g., external of the patient) to detect a fluid temperature and/or fluid pressure of irrigation fluid flowing towards the treatment site and the fluid being extracted from the treatment site. In other examples, the apparatus may additionally or alternatively comprise a fluid flow rate sensor disposed in one or each of the irrigation fluid paths and/or in the suction path.

Based on the detected temperature, pressure, and/or flow rate of the irrigation fluid and suction fluid, the fluid control system can control the irrigation fluid system and the suction system to balance the fluid flow rate towards, and away from, the treatment site.

Optionally, a safety valve is provided and configured to open above a predetermined pressure to prevent overpressure at the treatment site, for example within the kidney.

The fluid control system may optionally be configured to temporarily reverse the direction of flow of irrigation fluid in the or each irrigation fluid channel, and/or reverse the direction of flow in the suction channel. The fluid control system may reverse the direction of flow to dislodge blockages in an irrigation fluid channel or suction channel, or alter a fluid flow field at the treatment site to dislodge an object, for example a kidney stone.

According to a further aspect of the present disclosure, there is provided a method of endourology. The method comprises positioning an access sheath in a patient's urinary system, wherein the access sheath comprises an access channel for a surgical tool and an irrigation fluid channel. The method further comprises passing a surgical tool through the access channel of the access sheath to a treatment site, the surgical tool comprising a suction channel for removing fluid from the treatment site. The method further comprises conveying irrigation fluid to the treatment site through the irrigation fluid channel, and extracting fluid from the treatment site through the suction channel of the surgical tool.

In some examples, the method of endourology comprises a method of ureteroscopy, in which the access sheath is positioned within a patient's urethra, bladder, and ureter to provide access to the kidney. The surgical tool may be a ureteroscope.

In other examples, the endourology comprises a percutaneous nephrolithotomy (PCNL) procedure, where the access sheath is passed through a surgical opening formed in the patient's body and into the kidney to provide access for the surgical tool.

The method of endourology provides irrigation fluid and suction in an opposite direction to what is currently known. In particular, providing irrigation fluid via an irrigation fluid channel in the access sheath, and suction via a suction channel in a surgical tool, is a reverse of currently commercially available endourology apparatus. This reverse flow direction provides several advantages. In particular, during an endourological procedure using an endoscope or ureteroscope the reverse flow direction keeps debris away from any camera in the surgical tool. The reverse flow direction also tends to keep the kidney stone closer to the surgical tool, rather than pushing it away, which is advantageous for lithotripsy procedures and for procedures to inspect kidney stones. The reverse flow direction additionally ensures that debris is primarily entrained in irrigation fluid flowing towards the suction channel because the suction channel is aligned with the surgical tool and so aligned with any objects, such as kidney stones, that are being inspected, ablated, and/or extracted. Therefore, debris is not circulated around the kidney by inflowing irrigation fluid and can be more easily extracted.

In some examples, the method of endourology is a method of lithotripsy comprising using a medical laser to break a kidney stone apart at the treatment site.

In some examples, the method comprises varying a fluid flow field at the treatment site. In particular the method may comprise controlling conveyance of the irrigation fluid to the treatment site and/or extraction of fluid from the treatment site to vary a fluid flow field at the treatment site. Advantageously, such a method may provide for better operation of the surgical tool, for example to keep debris away from a camera lens of a ureteroscope or endoscope, or to dislodge or control the position of an object at the treatment site, for example a kidney stone. For example, a kidney stone may be lodged in the lower pole of the kidney or within pockets where the kidney medullary pyramids connect to the renal calyces. In examples, the operator may vary, or control, the speed and/or direction of the irrigation fluid flow at the treatment site to dislodge the kidney stone with the irrigation fluid.

In some examples, the access sheath comprises a plurality of irrigation fluid channels, and the method comprises conveying irrigation fluid to the treatment site via the plurality of irrigation fluid channels. In such examples, the method may further comprise selectively operating each of the plurality of irrigation fluid channels independently to vary a fluid flow field at the treatment site. In other such examples, the method may comprise controlling a pressure or flow rate of irrigation fluid in the plurality of irrigation fluid channels to vary a fluid flow field at the treatment site. In some examples, flow direction may be temporarily reversed to dislodge an object or clear debris.

In some examples, the one or more irrigation fluid channels are movable relative to the surgical tool (and suction channel), and the method comprises moving one or more of the irrigation fluid channels and the surgical tool to vary a fluid flow field at the treatment site. The method of moving one or more of the irrigation fluid channels and the surgical tool may comprise rotating the surgical tool or a part of the access sheath to alter an arrangement of the one or more irrigation fluid channels and suction channel at the treatment site.

In examples, the method further comprises controlling conveyance of irrigation fluid via the or each irrigation fluid channel and controlling a suction pressure generated in the suction channel to balance the flow rate of fluid towards, and away from, the treatment site.

In various examples, the method may further comprise receiving an operator input to vary one or more of: an irrigation fluid flow rate, an irrigation fluid pressure, or a suction pressure. The method may further comprise controlling conveyance of irrigation fluid through the or each irrigation fluid channel and controlling extraction of fluid through the suction channel according to the operator input and to balance the flow rate of fluid towards, and away from, the treatment site.

In examples where irrigation fluid is selectively provided to a plurality of irrigation fluid channels, then in response to changing a configuration of irrigation fluid channels (e.g., opening, closing, or changing a pressure or flow rate in one or more irrigation fluid channels) the method may comprise controlling the suction system to adjust the suction pressure in the suction channel accordingly.

The method may further comprise detecting one or more fluid properties of irrigation fluid flowing towards the treatment site, and/or fluid being extracted through the suction channel. The fluid property or properties may be a fluid temperature, a fluid pressure, and/or a fluid flow rate of the irrigation fluid or the fluid being extracted by the suction system. The fluid property may be detected at the treatment site, in an irrigation fluid path, and/or in a suction path.

Based on the detected temperature, pressure, and/or flow rate of the irrigation fluid and suction fluid, the method may control the irrigation fluid system and the suction system to balance the fluid flow rate towards, and away from, the treatment site.

The method may further comprise coupling the access sheath to the surgical tool as the surgical tool is passed through an access channel of the access sheath.

According to a further aspect of the present disclosure, there is provided a method of endourology comprising positioning an access sheath in a patient's urinary system, passing a surgical tool through a lumen of the access sheath to a treatment site, and coupling the access sheath to the surgical tool as the surgical tool is passed into the lumen of the access sheath during use.

In some examples, the method of endourology comprises a method of ureteroscopy, in which the access sheath is positioned within a patient's urethra, bladder, and ureter to provide access to the kidney. The surgical tool may be a ureteroscope.

In other examples, the endourology comprises a percutaneous nephrolithotomy (PCNL) procedure, where the access sheath is passed through a surgical opening formed in the patient's body and into the kidney to provide access for the surgical tool.

Once coupled, the method may comprise moving the surgical tool and access sheath together towards the kidney to position a distal end of the access sheath and a distal end of the surgical tool at or near the treatment site. Advantageously, irrigation fluid can be provided through the access sheath, and suction provided through the surgical tool, and the irrigation fluid and suction are provided at the treatment site to improve operability of the surgical tool.

Coupling the access sheath to the surgical tool is particularly advantageous for ureteroscopy procedures, where an access sheath is typically positioned in the patient's urethra, bladder and partly along the ureter. The coupling mechanism provides for moving the end of the access sheath into the kidney during use, as the ureteroscope is passed through the access sheath. Accordingly, the access sheath can be positioned at the treatment site.

The method may be configured to couple the access sheath to the surgical tool at or near the distal end of the access sheath and the distal end of the surgical tool.

In examples, a distal end portion of the access sheath may be flexible, and the method may further comprise articulating the surgical tool and the distal end portion of the access sheath.

The distal end portion of the access sheath may be extendible, and the method may further comprise extending the distal end portion of the access sheath by moving the surgical tool.

The method may further comprise decoupling the access sheath and the surgical tool.

### BRIEF DESCRIPTION OF THE DRAWINGS

Embodiments of the disclosure are further described hereinafter with reference to the accompanying drawings, in which:
FIG. 1A is schematic cross-sectional view of an access sheath and a ureteroscope during ureteroscopy;
FIG. 1B is an end view the access sheath and ureteroscope of FIG. 1A;
FIG. 2 is an end view of an example ureteroscope;
FIGS. 3A to 3E are end views of different example access sheaths with a ureteroscope;
FIGS. 4A to 4F are end views of further different example access sheaths with a ureteroscope, the access sheaths having a plurality of irrigation fluid channels;
FIG. 5A is a schematic cross-section of the distal end of the access sheath showing an insert in the access sheath;
FIG. 5B is an end view of the access sheath, insert and ureteroscope of FIG. 5A;
FIG. 6A is a schematic view of apparatus for ureteroscopy, including an access sheath, an adapter, and a ureteroscope;
FIG. 6B is a cross-sectional view of the adapter of FIG. 5A;
FIG. 7A is a schematic view of apparatus for ureteroscopy, including an access sheath with a plurality of irrigation fluid channels, an adapter, and a ureteroscope;
FIG. 7B is a cross-sectional view of the adapter of FIG. 6A;
FIG. 8 is schematic view of apparatus for ureteroscopy that includes one or more sensors and a fluid control system;
FIGS. 8A to 9B illustrate opening and closing of irrigation fluid channels in the access sheath to vary a fluid flow field at the treatment site;
FIGS. 10A and 10B illustrate rotation of the ureteroscope within the access sheath to vary a fluid flow field at the treatment site, where the access sheath has a single irrigation fluid channel;
FIGS. 11A and 11B illustrate rotation of the ureteroscope within the access sheath to vary a fluid flow field at the treatment site, where the access sheath has a plurality of irrigation fluid channels;
FIGS. 12A and 12B illustrate relative movement of the ureteroscope and one or more irrigation fluid channels within the access sheath to vary a fluid flow field at the treatment site;
FIG. 13 illustrates an access sheath and ureteroscope positioned within a patient's kidney, in particular within the renal pelvis;
FIGS. 14A and 14B illustrate a first example coupling mechanism for coupling the ureteroscope to the access sheath during use;
FIGS. 15A and 15B illustrate a further example coupling mechanism for coupling the ureteroscope to the access sheath during use;
FIGS. 16A and 16B illustrate a further example coupling mechanism for coupling the ureteroscope to the access sheath during use;
FIGS. 17A and 17B illustrate a further example coupling mechanism for coupling the ureteroscope to the access sheath during use;
FIG. 18 illustrates use of an access sheath in a percutaneous nephrolithotomy procedure;
FIG. 19 illustrates a method of ureteroscopy that includes providing irrigation fluid via an irrigation fluid channel in the access sheath and extracting fluid via a suction channel in the ureteroscope; and
FIG. 20 illustrates a method of ureteroscopy that includes coupling the ureteroscope to the access sheath.

### DETAILED DESCRIPTION

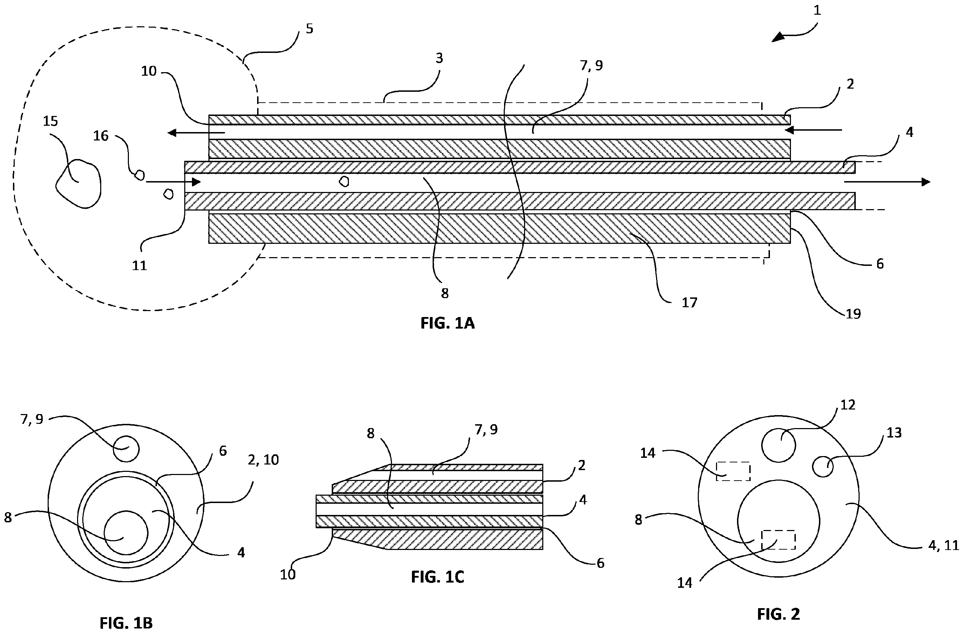

FIG. 1A illustrates a ureteroscopy procedure using apparatus for ureteroscopy. FIG. 1A shows an access sheath 2 positioned in a urinary system 3 of a patient and a surgical tool 4 passing through the access sheath 2 to a treatment site 5, in particular a kidney of the patient. The access sheath 2 is passed through the urethra, bladder, and ureter to the kidney. The access sheath 2 has an access channel for receiving the surgical tool. In this example the access sheath has a ureteroscope channel 6 for receiving a ureteroscope 4. As illustrated, the ureteroscope 4 passes through the ureteroscope channel 6 to the kidney during ureteroscopy.

In the example of FIG. 1A, the access sheath 2 and ureteroscope 4 are sized appropriately for ureteroscopy. In particular, ureteroscopes typically have an outer diameter that may vary between 8 and 9.5 French, although some ureteroscopes may be smaller or larger. An access sheath for ureteroscopy typically has an outer diameter of between 12 and 15 French and a working channel diameter of between 10 and 13 French, although smaller and larger access sheaths are possible. The access sheath 2, in particular the ureteroscope channel 6, is sized to accommodate the ureteroscope 4. Typically, the access sheath 2 has tapered or rounded edges, particularly at the distal end 10, for smooth passage through the patient's urethra, bladder, and ureter. FIG. 1C illustrates a tapered distal end 10 of the access sheath 2, although it will be appreciated that additionally or alternatively the edges may be rounded.

Dimensions of the access sheath 2, ureteroscope 4 and patient's urinary system 3 are exaggerated in the Figures for clarity.

The access sheath 2 is generally an elongate tube having a cylindrical outer form for passing through the patient's urinary system 3. In addition, the ureteroscope 4 has an elongate scope portion, described further below, that generally has a cylindrical form. The elongate scope portion may have the ability to articulate e.g. bend up or down. The elongate scope portion may be able to articulate in excess of 270 degrees. The elongate scope portion may have good torsional strength that allows the surgeon to move the distal end of the ureteroscope. The ureteroscope may also contain a connector or connectors for providing video and power connections between a control system and a camera and/or light in the ureteroscope.

FIG. 1B shows a view of the distal end 10 of the access sheath 2 and ureteroscope 4. As shown in FIGS. 1A and 1B, the access sheath 2 additionally comprises an irrigation fluid channel 7. The ureteroscope 4 comprises a suction channel 8. In use, as illustrated, irrigation fluid is passed through the irrigation fluid channel 7 to the treatment site 5, and fluid is extracted via the suction channel 8 of the ureteroscope 4. In this example the irrigation channel 7 is formed by a second lumen 9 in the access sheath 2, distinct from the ureteroscope channel 6. As shown in FIGS. 1A and 1C, the irrigation fluid channel 7 may have an outlet at the distal end 10 of the access sheath that may be in a taper or may be a planar end face at the distal end 10 of the access sheath.

FIG. 2 shows a view of the distal end 11 of an example ureteroscope 4 that includes the suction channel 8, a camera 12 and a light 13. The distal end 11 is positioned at the treatment site 5 during use, as shown in FIG. 1A. The light 13 illuminates the treatment site 5 and the camera 12 provides images and/or video to an operator of the ureteroscope 4.

For lithotripsy a laser fibre 14 may be incorporated into the ureteroscope 4 or passed through the suction channel 8 (otherwise termed a working channel), as illustrated in FIG. 2. The laser fibre 14 is operated to ablate objects 15 in the kidney, such as kidney stones as shown in FIG. 1A. Ablation of the kidney stones 15 breaks them into smaller particles, such as debris 16, which is extracted through the suction channel 8 entrained in fluid.

The irrigation fluid entrains the debris 16 to ease suction extraction. In addition, the irrigation fluid flow between the irrigation fluid channel 7 and the suction channel 8 acts to keep debris away from the camera 12 and light 13, ensuring that the operator's view remains unobscured. Irrigation fluid is also provided to reduce fluid temperature increases in the kidney that may be caused by operation of the laser fibre 14.

Advantageously, the inventors have found that providing an irrigation fluid channel 7 in the access sheath 2 and a suction channel 8 in the ureteroscope 4 generates beneficial irrigation fluid flow at the treatment site 5. In particular, it is normal to provide irrigation fluid via a working channel of a ureteroscope and passive fluid outflow or active suction extraction through via the access sheath (typically in the space surrounding the ureteroscope). However, the inventors have found that reversing the flow direction of the irrigation fluid, as illustrated in FIG. 1A, is beneficial for keeping debris away from the camera 12 and light 13. In addition, as the suction channel 8 is provided in the ureteroscope 4, in some implementations the suction channel 8 is always positioned advantageously for extraction of debris 16 as it is facing the object being treated, for example the kidney stone being ablated.

In the example FIGS. 1A and 1B the ureteroscope channel 6 and the irrigation fluid channel 7 are separate lumens formed in the access sheath 2. In particular, as illustrated, the access sheath 2 has an elongate body 17 and the ureteroscope channel 6 and the irrigation fluid channel 7 each comprise a lumen 18, 9 extending through the elongate body 17 to the distal end 10 of the access sheath 2 (i.e., to the treatment site 5 during use). At a proximal end 19 of the access sheath 2, external of the patient during use, the ureteroscope channel 6 and the irrigation fluid channel 7 can be separately accessed and/or connected to further apparatus, such as an adapter, irrigation fluid system, and a suction system as described further hereinafter.

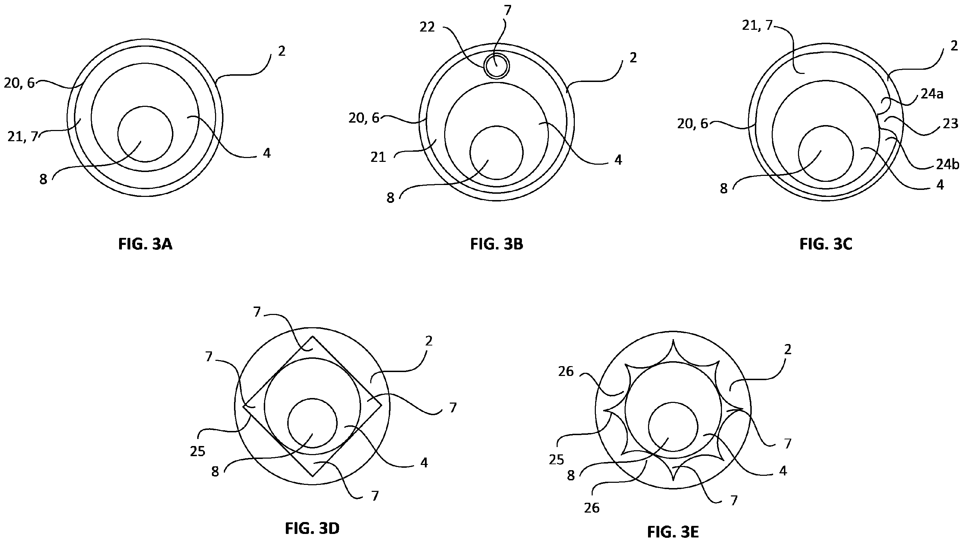

FIGS. 3A to 3E Illustrate different example access sheaths 2, each having a ureteroscope channel 6 and an irrigation fluid channel 7.

In the example of FIG. 3A, the access sheath 2 comprises one lumen 20 through which the ureteroscope 4 is passed. The lumen 20 is larger than the ureteroscope 4, providing an annular region 21 that acts as the irrigation fluid channel 7. The lumen 20 acts as the ureteroscope channel 6 and irrigation fluid channel 7.

In the example of FIG. 3B, the access sheath 2 comprises one lumen 20 through which the ureteroscope 4 is passed. The lumen 20 defines the ureteroscope channel 6. The lumen 20 is larger than the ureteroscope 4, providing an annular region 21 within which a separate tube 22 is provided. The lumen 22 defines the irrigation fluid channel 7.

In the example of FIG. 3C, the access sheath 2 comprises one lumen 20 through which the ureteroscope 4 is passed. The lumen 20 provides the ureteroscope channel 6. The lumen 20 is larger than the ureteroscope 4 and comprises a sidewall protrusion 23 that constrains the position of the ureteroscope 4 within the lumen 20. The irrigation fluid channel 7 is defined in the space 21 between the ureteroscope 4 and the sidewall of the lumen 20. In this example, unlike in the example of FIG. 3A, the sidewall protrusion 23 ensures that irrigation fluid can always flow through a defined part of the lumen 20 because the ureteroscope 4 cannot move into and block the regions 24a, 24b adjacent to the sidewall protrusion 23.

In the examples of FIG. 3D and 3E the access sheath 2 comprises one lumen 25 having a non-circular cross-sectional shape. In the example of FIG. 3D the lumen 25 comprises a square shape. In the example of FIG. 3E the lumen 25 comprises a plurality of sidewall protrusions 26, in this example rounded sidewall protrusions 26. In both of these examples the position of the ureteroscope 4 within the lumen 25 is constrained by the non-circular cross-sectional shape of the lumen 25, and the irrigation channel 7 is provided in the space(s) between the ureteroscope 4 and the sidewall of the lumen 25.

As illustrated in FIGS. 3A to 3E, the suction channel 8 of the ureteroscope 4 may be eccentrically positioned within the ureteroscope 4 - i.e., not in the centre of the ureteroscope 4. Accordingly, rotation of the ureteroscope 4 within the access sheath 2 changes the relative positions of the suction channel 8 and the or each irrigation fluid channel 7. Therefore, rotation of the ureteroscope 4 within the access sheath 2 can vary the irrigation fluid flow field between the or each irrigation fluid channel 7 and the suction channel 8 at the treatment site 5.

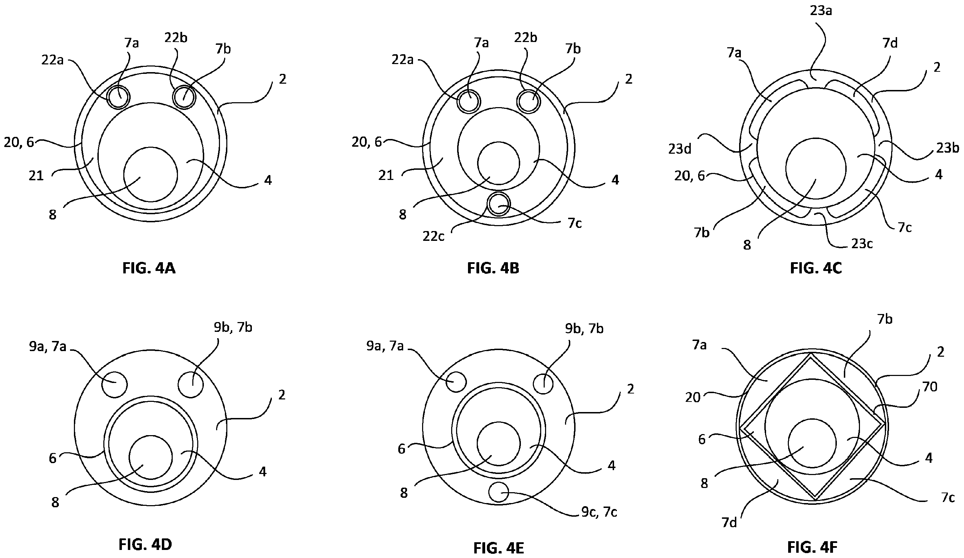

FIGS. 4A to 4F illustrate further example access sheaths 2, each having a ureteroscope channel 6 and a plurality of irrigation fluid channels 7a, 7b, (7c, ...).

In the example of FIG. 4A the access sheath 2 comprises one lumen 20 through which the ureteroscope 4 is passed. The lumen 20 provides the ureteroscope channel 6. The lumen 20 is larger than the ureteroscope 4, providing an annular region 21 within which two separate tubes 22a, 22b are provided, the two tubes 22a, 22b defining two different irrigation fluid channels 7a, 7b. The example of FIG. 4B is the same as FIG. 4A except that three tubes 22a, 22b, 22c are provided in the annular region 21, providing three irrigation fluid channels 7a, 7b, 7c. It will be apparent that any number of tubes 22 could be provided in the annular region 21 to provide any number of suction channels 7.

In the example of FIG. 4C the access sheath 2 comprises one lumen 20 through which the ureteroscope 4 is passed. The lumen 20 provides the ureteroscope channel 6. The lumen 20 is larger than the ureteroscope 4 and comprises a plurality of sidewall protrusions 23a-23d that sealingly engage the ureteroscope 4. A plurality of irrigation fluid channels 7a-7d, in this example four irrigation fluid channels 7a-7d, are provided in the spaces within the lumen 20 and between the ureteroscope 4 and the sidewall protrusions 23a-23d. It will be appreciated that any number of two or more sidewall protrusions 23 could be provided to create two or more irrigation fluid channels 7.

The sidewall protrusions 23a-23d are configured to engage, and seal against, the ureteroscope 4. It will be appreciated that the sealing engagement between the sidewall protrusions 23a-23d and the ureteroscope 4 may merely be an abutment of the ureteroscope 4 against the sidewall protrusions 23a-23d. In use, pressure differences between irrigation fluid in the different irrigation fluid channels 7a-7d will not be significant, so the sealing engagement can be quite simple. The sidewall protrusions 23a-23d may comprise a resiliently deformable material, and/or the access sheath 2 itself may comprise a resiliently deformable material, and the sidewall protrusions 23a-23d may be configured to provide a sliding fit for the ureteroscope 4 while still maintaining some level of sealing between adjacent irrigation fluid channels 7a-7d.

In a similar example, the access sheath 2 also has a membrane arranged between the ureteroscope 4 and the access sheath 2 within the lumen 20. The membrane may be attached to each of the sidewall protrusions 23a-23d and the irrigation fluid channels 7a-7d are defined between the membrane and the lumen 20. The membrane can ensure that the irrigation fluid channels 7a-7d are sealed from each other and from the ureteroscope channel 6.

In the example of FIG. 4D two separate irrigation fluid channels 7a, 7d are defined in the access sheath 2. In particular, the access sheath 2 has a ureteroscope channel 6 for the ureteroscope 4, and two separate lumens 9a, 9b extending through the access sheath 2 to provide irrigation fluid channels 7a, 7b. The example of FIG. 4E is similar to the example of FIG. 4D, with three separate lumens 9a, 9b, 9c defining three irrigation fluid channels 7a, 7b, 7c. It will be appreciated than any number of lumens 9 can be provided to define a corresponding number of irrigation fluid channels 7.

In the example of FIG. 4F, the access sheath 2 comprises a lumen 20 and a deformable membrane 70 that is attached to the access sheath 2 within the lumen 20 at two or more locations about the circumference of the lumen 20. The deformable membrane defines the ureteroscope channel 6, and also delimits a plurality of irrigation fluid channels 7a, 7b, 7c, 7d between the deformable membrane 70 and the sidewall of the lumen 20. The deformable membrane 70 provides a ureteroscope channel 20 that is sealed from the irrigation fluid channels 7a, 7b, 7c, 7d, and the attachment of the deformable membrane 70 to lumen 20 also seals the irrigation fluid channels 7a, 7b, 7c, 7d from each other.

In all of the examples of FIG. 1B, FIGS. 3A to 3E, and FIGS. 4A to 4F the access sheath 2 provides one or more irrigation fluid channels 7 for conveying irrigation fluid to the treatment site (5, see FIG. 1A). As illustrated in FIGS. 4A to 4F, the suction channel 8 of the ureteroscope 4 may be eccentrically positioned within the ureteroscope 4 - i.e., not in the centre of the ureteroscope 4. Accordingly, rotation of the ureteroscope 4 within the access sheath 2 changes the relative positions of the suction channel 8 and the plurality of irrigation fluid channels 7. Therefore, rotation of the ureteroscope 4 within the access sheath 2 can vary the irrigation fluid flow field between each irrigation fluid channel 7 and the suction channel 8 at the treatment site 5.

In additional examples, rotation of the ureteroscope 4 within the ureteroscope channel 6 may open and close at least some of the plurality of irrigation fluid channels 7.

In some examples an outer profile of the access sheath is cylindrical, but in alternative examples the outer profile of the access sheath may be non-cylindrical, for example generally square or generally triangular. The outer profile of the access sheath may match an inner profile of a lumen through the access sheath, for example a lumen 20 that provides the access channel 6 for the surgical tool as illustrated in FIG. 3D.

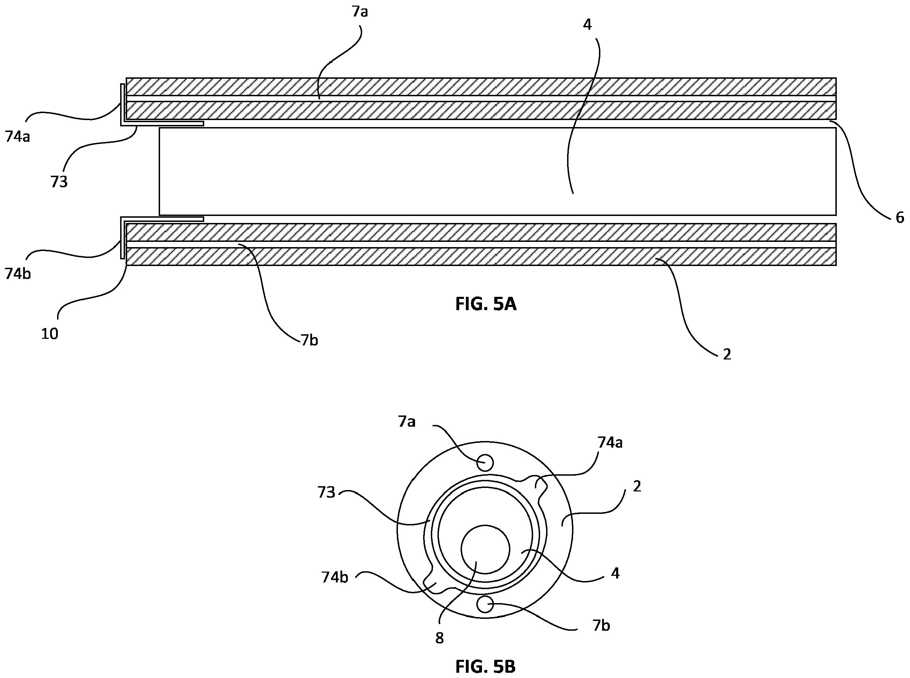

FIGS. 5A and 5B show an example where the access sheath 2 includes an insert 73 at the distal end 10 that is engaged by the ureteroscope 4. The insert 73 is rotatable relative to the access sheath 2 and comprises one or more blocking members 74a, 74b arranged to open and close the irrigation fluid channels 7a, 7b in the access sheath 2. The ureteroscope 4 may rotationally couple to the insert 73, for example by splines and grooves or other rotationally corresponding features.

As shown in FIG. 5B, rotation of the ureteroscope 4 and the insert 73 moves the blocking members 74a, 74b into and out of alignment with the irrigation fluid channels 7a, 7b. The insert 73 may comprise blocking members 74a, 74b arranged to be positioned such that one or more of the irrigation fluid channels 7a, 7b is open while one or more other irrigation fluid channels 7a, 7b is closed.

Partial occlusion of one or more irrigation fluid channels 7a, 7b allows control of irrigation fluid flow rate(s) at the treatment site without having to vary the input of irrigation fluid at the proximal end of the access sheath 2.

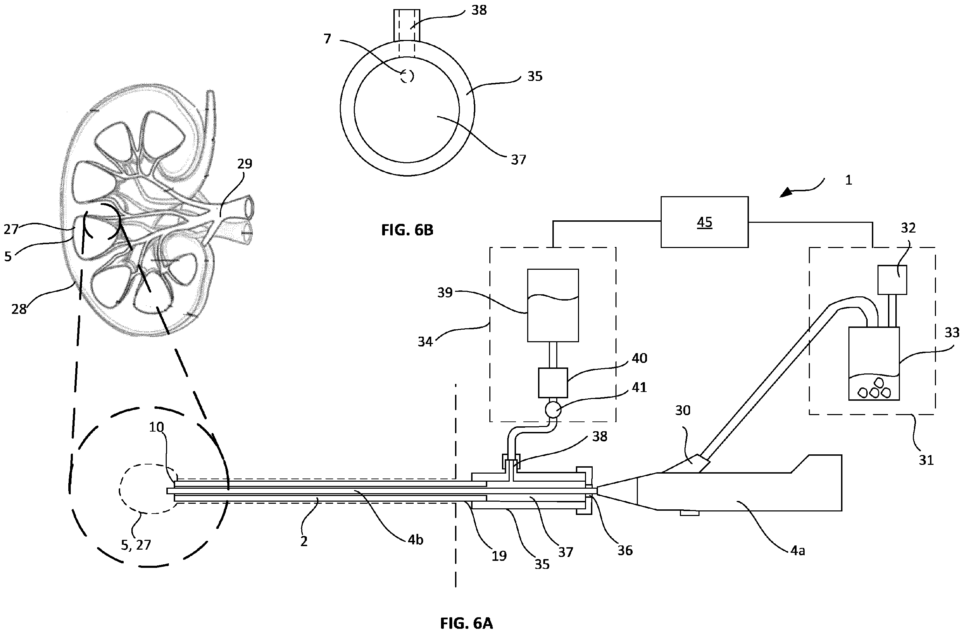

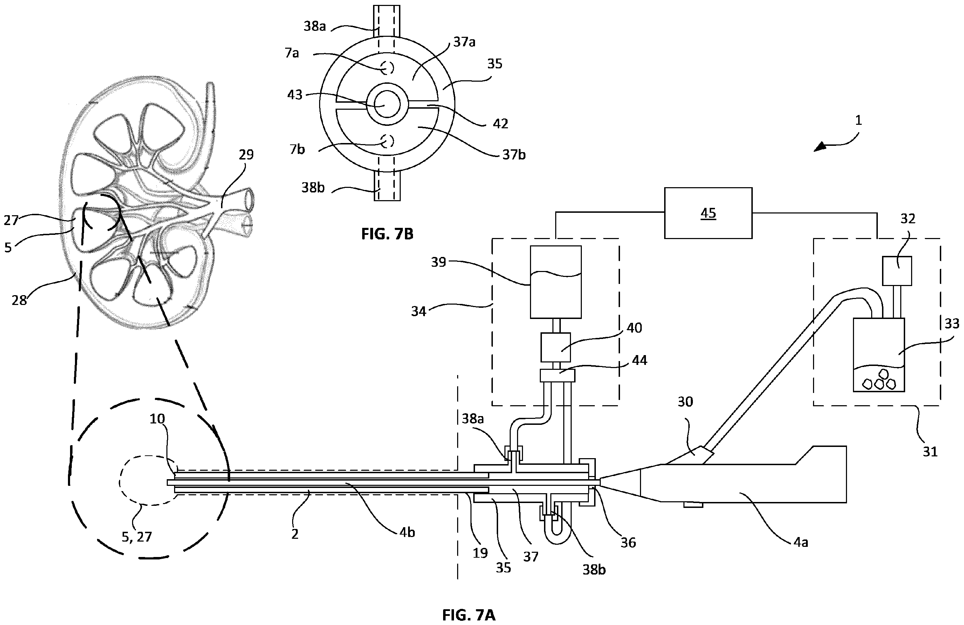

FIGS. 6A, 6B, 7A and 7B schematically illustrate apparatus for ureteroscopy 1 using the access sheath 2 and ureteroscope 4 described above.

As shown in FIGS. 6A and 7A, during use the access sheath 2 is positioned within the urinary system 5 of a patient. In particular, the access sheath 2 is positioned within the urethra, bladder, and ureter of the patient. In some examples, the distal end 10 of the access sheath 2 is positioned in the ureter, or in the renal pelvis 29 during use. However, in some examples, as described further hereinafter, during use the distal end 10 of the access sheath 2 is positioned in the kidney 28, in particular in a calyx 27 of the kidney. A calyx 27 of the kidney 28 is shown schematically in FIGS. 6A and 7A, and the distal end 10 of the access sheath 2 is located in the calyx 27.

A proximal end 19 of the access sheath 2 remains external of the patient.

As shown in FIGS. 6A and 7A, the apparatus 1 includes a ureteroscope 4. The ureteroscope 4 includes an operator portion 4a and an elongate scope portion 4b. The scope portion 4b is as described with reference to FIG. 2, and during use is passed through the access sheath 2 to the treatment site 5. The scope portion 4b extends from the operator portion 4a, which provides the relevant connections and controls for operating the ureteroscope 4.

As illustrated in FIGS. 6A and 7A, the operator portion 4a also comprises a suction port 30 that is in in fluid communication with the suction channel (8, see FIG. 1A) of the ureteroscope 4. The suction port 30 is connected to a suction system 31 that generates suction pressure in the suction port 30 and suction channel (8, see FIG. 1A) in the ureteroscope 4 for extracting fluid and debris from the treatment site 5.

As shown in FIGS. 6A and 7A, the suction system 31 comprises a suction source 32, which may be a suction pump (e.g., a vacuum pump), or it may be a connection to a general suction source, for example a building suction source. Alternatively, the suction source 32 may comprise a venturi arrangement for generating suction pressure from a compressed flow of fluid, for example compressed air. In this example, the suction system 31 may comprise a pump or compressor for generating a compressed fluid flow through the venturi arrangement, and suction generated by the venturi arrangement is provided to the suction port 30 and suction channel (8, see FIG. 1A) in the ureteroscope 4. Alternatively, the suction system 31 may connect to a source of compressed fluid, such as a compressed air reservoir or building system.

As shown in FIGS. 6A and 7A, the suction system 31 also includes a collection chamber 33 disposed between the suction port 30 and the suction source 32. The collection chamber 33 acts to collect fluid and debris removed from the treatment site 5 during use. It will be appreciated that fluid and debris may alternatively be removed from the suction path in other ways, for example using a filter or similar.

The suction system 31 may comprise one or more valves, such as a bleed valve, to control the suction pressure applied to the suction channel (8, see FIG. 1A) of the ureteroscope 4. For example, a bleed valve may be positioned between the suction port 30 of the ureteroscope 4 and the suction system 31. Bleeding suction pressure by opening the bleed valve would reduce the suction pressure (i.e., an increase in actual pressure) generated in the suction channel (8, see FIG. 1A).

An operator may manually control or adjust the suction system 31 to adjust the rate of extraction of fluid and debris from the treatment site 5.

As also shown in FIGS. 6A and 7A, the apparatus 1 includes an irrigation fluid system 34 for supplying irrigation fluid to the or each irrigation channel (7, see FIG. 1A) in the access sheath 2.

In these examples, the irrigation fluid system 34 connects to the access sheath 2 via an adapter 35, shown in FIGS. 6A and 7A. The adapter 35 connects to a proximal end 19 of the access sheath 2, external of the patient. The adapter 35 has an opening 36 for receiving the scope portion 4b of the ureteroscope 4. The opening 36 may include a seal configured to seal against the scope portion 4b. Alternatively, the operator portion 4a of the ureteroscope 4 may also attach to the adapter 35. The scope portion 4b of the ureteroscope 4 passes through the adapter 35 and the access sheath 2 to the treatment site 5, as previously described. In this way, the adapter 35 defines a sealed internal chamber 37 that is in fluid communication with the or each irrigation fluid channel (7, see FIG. 1A) in the access sheath 2.

In the example of FIGS. 6A and 7B, the adapter 35 comprises an inlet 38 for connection to an irrigation fluid system 34. The inlet 38 is in fluid communication with the sealed internal chamber 37 of the adapter 35. As illustrated, the inlet 38 is connected to the irrigation fluid system 34. The irrigation fluid system 34 has a source of irrigation fluid 39, for example a reservoir of irrigation fluid. In some examples, the irrigation fluid is gravity-fed to the inlet 38 to provide irrigation fluid to the adapter 35 and the or each irrigation fluid channel (7, see FIG. 1A) of the access sheath 2. Alternatively, the irrigation fluid system 34 may comprise a pump 40 arranged to pump irrigation fluid from the reservoir 39 to the inlet 38. In these examples, the irrigation fluid system 34 may include a valve 41 disposed between the reservoir 39 and the inlet 38 to control flow of irrigation fluid. The irrigation fluid system 34 may additionally comprise a safety valve configured to open and vent irrigation fluid at a safety threshold pressure, to prevent overpressure in the kidney.

FIG. 6B illustrates a cross-section of the adapter 35, also showing the location of the irrigation fluid channel 7 in the access sheath 2.

It will be appreciated that the adapter 35 described with reference to FIGS. 6A and 6B may be used with any of the example access sheaths 2 described with reference to FIGS. 2, or 3A to 4F. If the adapter described with reference to FIGS. 6A and 6B is used with an access sheath 2 having more than one irrigation fluid channel 7 then the irrigation fluid is provided to all of the irrigation fluid channels 7 at substantially equal pressure via the chamber 37 of the adapter 35.

In alternative examples, the adapter 35 may include one or more valves for closing/opening fluid connections to the irrigation fluid channels 7. The valves may be actuated by a button or knob on an outer surface of the adapter 35.

In the example of FIGS. 7A and 7B, the adapter has two inlets 38a, 38b for providing irrigation fluid to the sealed internal chamber 37 and to the irrigation fluid channels (7, see FIG. 1A). FIG. 7B shows a cross-section of the adapter 35, and as illustrated the adapter 35 comprises a divider 42 extending across the internal chamber 37 of the adapter 35 and dividing the internal chamber 37 into two sub-chambers 37a, 37b. The divider 42 also comprises a path 43 for the ureteroscope 4. As illustrated, the first sub-chamber 37a is in fluid communication with a first irrigation fluid channel 7a of the access sheath 4, and the second sub-chamber 37b is in fluid communication with a second irrigation fluid channel 7b of the access sheath 4. A first inlet 38a is provided for the first sub-chamber 37a, and a second inlet 38b is provided for the second sub-chamber 37b. The first and second inlets 38a, 38b are both connected to the irrigation fluid system 34, which comprises a control mechanism 44 for selectively supplying irrigation fluid to the first inlet 38a and/or the second inlet 38b. The control mechanism 44 may additionally be adapted to provide irrigation fluid to the first inlet 38a and/or the second inlet 38b at the same or different pressures or flow rates. Accordingly, the irrigation fluid system 34 can control supply of irrigation fluid to the first irrigation fluid channel 7a and/or second irrigation fluid channel 7b in the access sheath 2.

The control mechanism 44, for controlling supply of irrigation fluid to the first inlet 38a and/or second inlet 38b of the adapter 35 may comprise a plurality one or more valves or a similar arrangement, for example a rotatable component that crimps one or more deformable fluid conduits (e.g., tubes) connecting the irrigation fluid system 34 and the adapter 35.

In other examples, particularly if the access sheath 2 has more than two separate irrigation fluid channels 7, the adapter 35 may comprise additional sub-chambers 37c, (37d ...) in fluid communication with an irrigation fluid channel 7c, (7d ...) and the irrigation fluid system 34 so that irrigation fluid can be selectively provided to one or more of the plurality of irrigation fluid channels 7.

As illustrated in FIGS. 6A and 7A, the apparatus for ureteroscopy 1 further includes a fluid control system 45 configured to control both the irrigation fluid system 34 and the suction system 31. By controlling both the irrigation fluid system 34 and the suction system 31 the fluid control system 45 can control a flow rate of irrigation fluid through the treatment site 5. The fluid control system 45 may be configured to control the suction system 31 in dependence on operation of the irrigation fluid system 34, or it may be configured to control the irrigation fluid system 34 in dependence on operation of the suction system 31.

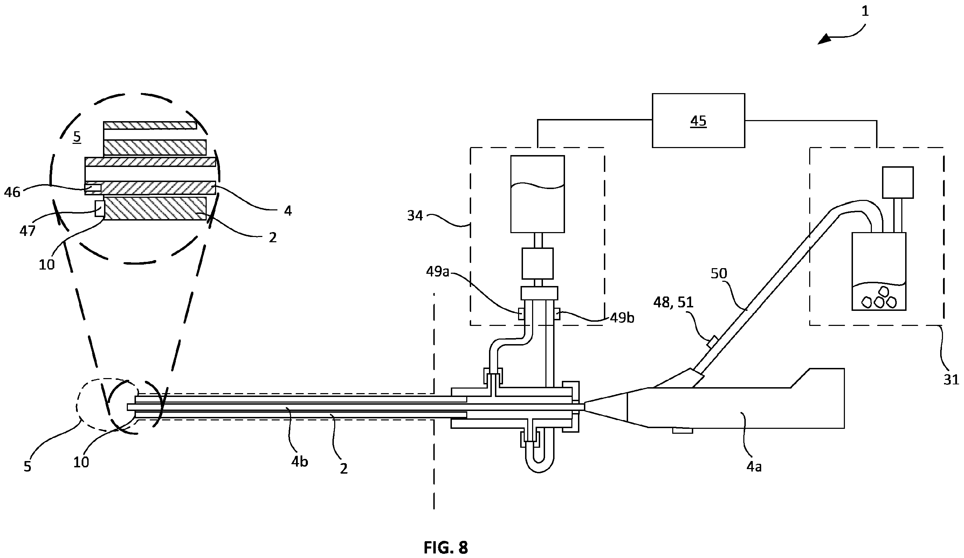

FIG. 8 schematically illustrates the fluid control system 45 of the apparatus for ureteroscopy 1. As shown, the ureteroscope 4 and/or the access sheath 2 and/or the irrigation fluid system 34 and/or the suction system 31 may comprise one or more sensors 46, 47, 48, 49 arranged to detect a fluid property.

In particular, a temperature and/or pressure sensor 46 may be disposed at the distal end 11 of the ureteroscope 4, and/or a temperature and/or pressure sensor 47 may be disposed at the distal end 10 of the access sheath 2. The or each temperature and/or pressure sensor 46, 47 is configured to detect a temperature and/or pressure of fluid at the treatment site 5. The or each temperature and/or pressure sensor 46, 47 is in communication with the fluid control system 45, which may be configured to control operation of the irrigation fluid system 34 and/or suction system 31 based on sensor signals received from the or each temperature and/or pressure sensor 46, 47. For example, in response to an increase in temperature at the treatment site 5, for example as a result of laser lithotripsy, the fluid control system 45 may increase a flow rate of the irrigation fluid and simultaneously increase the suction provided by the suction system 31 to increase the fluid flow rate of fluid at the treatment site 5. In another example, in response to an increase in fluid pressure at the treatment site 5 the fluid control system 45 may reduce the flow rate of irrigation fluid provided by the irrigation fluid system 34 and/or increase the suction provided by the suction system 31.

In another example, a temperature and/or pressure sensor 48 may additionally or alternatively be provided in the suction path to detect a temperature and/or pressure of fluid extracted from the treatment site 5. The temperature and/or pressure sensor 48 may be arranged, for example as illustrated, in the connection 50 between the ureteroscope 4 and the suction system 31. Alternatively the temperature and/or pressure sensor 48 may be arranged at the suction port 30 of the ureteroscope 4, or in the suction channel 8 of the ureteroscope 4. The temperature and/or pressure sensor 48 is in communication with the fluid control system 45, which may be configured to control operation of the irrigation fluid system 34 and/or suction system 31 based on sensor signals received from the temperature and/or pressure sensor 48. For example, in response to an increase in temperature of the fluid extracted from the treatment site 5, for example as a result of laser lithotripsy, the fluid control system 45 may increase a flow rate of the irrigation fluid and simultaneously increase the suction provided by the suction system 31 to increase the irrigation fluid flow rate at the treatment site 5.

In another example, a flow rate sensor 49a, 49b may be provided for the or each irrigation fluid channel 7. The flow rate sensor 49a, 49b may be arranged to detect a flow rate of irrigation fluid being provided to the or each irrigation fluid channel 7. As illustrated, the or each flow rate sensor 49a, 49b may be arranged in the irrigation fluid system 34 to measure a flow rate of irrigation fluid being provided to the or each irrigation fluid channel 7 in the access sheath 2.

Additionally or alternatively, a flow rate sensor 51 may be provided in a suction path of the apparatus 1 to detect a volumetric flow rate of fluid being extracted from the treatment site 5 by the suction system 31. The flow rate sensor 51 may be arranged, for example as illustrated, in the connection 50 between the ureteroscope 4 and the suction system 31. Alternatively the flow rate sensor 51 may be arranged at the suction port 30 of the ureteroscope 4, or in the suction channel 8 of the ureteroscope 4.

The or each flow rate sensor 49a, 49b, 51 is in communication with the fluid control system 45, which may be configured to control operation of the irrigation fluid system 34 and/or suction system 31 based on sensor signals received from the or each flow rate sensor 49a, 49b, 51. For example, in response to a decrease in volumetric flow rate in the suction path the fluid control system 45 may be configured to reduce a volumetric flow rate of irrigation fluid being provided to the or each irrigation fluid channel 7 to prevent overpressure at the treatment site 5.

Accordingly, the fluid control system 45 is configured to control flow of fluid to and from the treatment site 5 in dependence of each other and on detected fluid characteristics (e.g., temperature, pressure, flow rate).

In some examples, the fluid control system 45 is configured to control the irrigation fluid system 34 and the suction system 31 to balance irrigation fluid flow towards the treatment site 5 with fluid flow in the suction system 31. Accordingly, overpressure at the treatment site 5 is prevented and steady fluid flow through the treatment site 5 is maintained.

In some examples, the fluid control system 45 is configured to control the irrigation fluid system 34 and the suction system 31 such that fluid is extracted from the treatment site 5 via the suction channel 8 in the ureteroscope 4 at a marginally greater rate than irrigation fluid is provided to the treatment site 5 via the or each irrigation fluid channel 7 in the access sheath. For example, the volumetric flow rate of fluid extraction may be approximately 0.5% to 5% higher than the volumetric flow rate of irrigation fluid being provided to the treatment site 5. Such an arrangement accounts for urine production in the kidney, and prevents overpressure at the treatment site.

In some examples, the irrigation fluid system 34 and/or the suction system 31 comprises an emergency release valve operable to vent irrigation fluid or suction pressure external of the patient. The fluid control system 45 may be configured to automatically operate the or both emergency release valves if a detected fluid characteristic exceeds a safety threshold value. Such a situation may occur, for example, if the suction channel 8 of the ureteroscope 4 becomes blocked, which may lead to overpressure at the treatment site.

In some examples, the irrigation fluid system 34 comprises a manual control input for an operator to adjust a flow rate or pressure of the irrigation fluid in the or each irrigation fluid channel 7 of the access sheath 2. In this example, the fluid control system 45 may be configured to control the suction system 31 to adjust the suction pressure to correspond to the adjusted irrigation fluid flow rate or pressure.

In some examples, the suction system 31 comprises a manual control input for an operator to adjust a pressure in the suction channel 8 of the ureteroscope 2. In this example, the fluid control system 45 may be configured to control the irrigation fluid system 34 to adjust the irrigation fluid flow rate or pressure to correspond to the adjusted suction pressure.

As described with reference to FIGS. 4A to 4F, FIGS. 5A and 5B, and 7A and 7B, the apparatus 1 provides for independent control of a plurality of irrigation fluid channels 7a, 7b in the access sheath 2. Accordingly, the operator and/or flow control system 45 can control which irrigation fluid channels 7a, 7b are in operation, and at what pressure or volumetric rate of irrigation fluid. This provides for varying a fluid flow field at the treatment site 5.

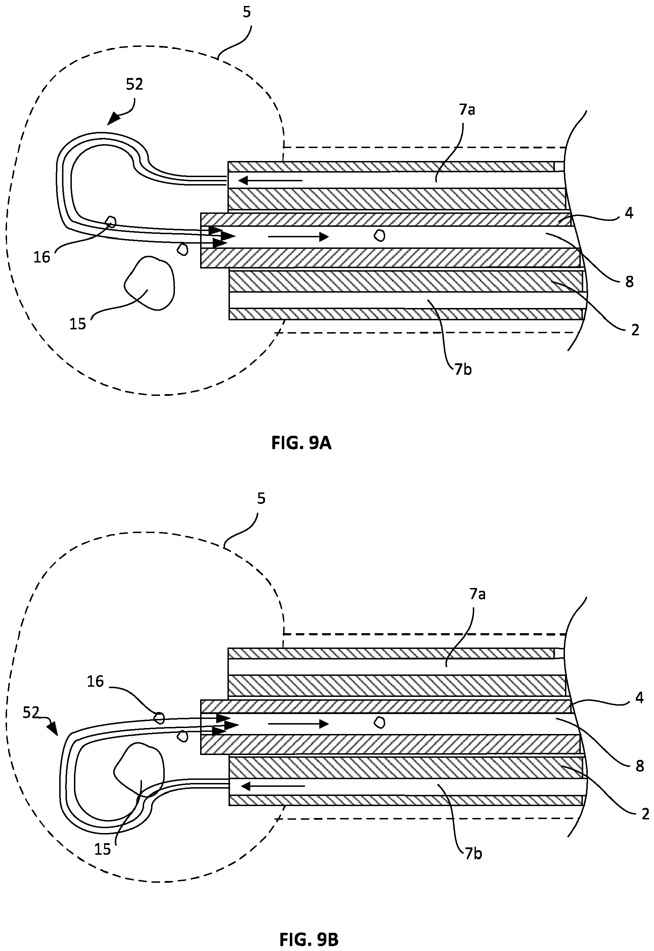

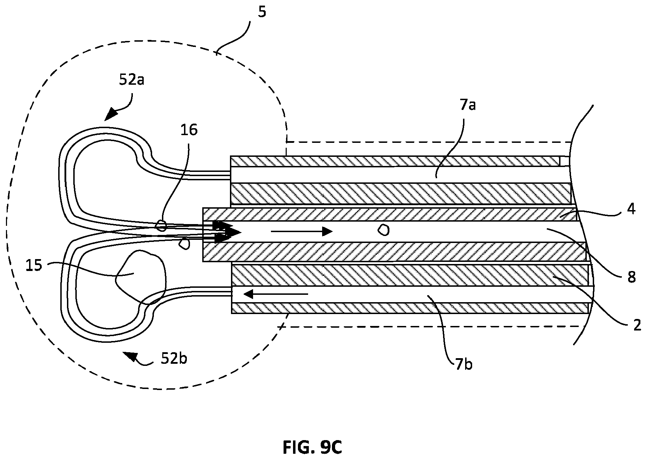

FIGS. 9A, 9B, and 9C illustrate how the fluid flow field at the treatment site 5 may be varied or controlled by selectively operating one or more of a plurality of irrigation fluid channels 7a, 7b. In the examples illustrated, the access sheath 2 comprises a first irrigation fluid channel 7a and a second irrigation fluid channel 7b, and, as per the other examples, the ureteroscope 4 comprises a suction channel 8.

In the illustrated example of FIG. 9A the first irrigation fluid channel 7a is in operation and irrigation fluid is flowing into the treatment site 5 from the first irrigation fluid channel 7a. The second irrigation fluid channel 7b is closed or turned off, so no irrigation fluid flows into the treatment site 5 from the second irrigation fluid channel 7b. Suction pressure is applied to the suction channel 8 or the ureteroscope and fluid is being extracted from the treatment site 5 via the suction channel 8. As shown, a fluid flow field 52 is generated between the first irrigation fluid channel 7a and the suction channel 8 at the treatment site 5. During use of the ureteroscope 4, for example during lithotripsy, an obstruction, for example a part of a kidney stone 15 or debris 16, may not be within the fluid flow field 52 and so may not be accessible to the ureteroscope 4. Additionally, the particular fluid flow field in the treatment site may cause issues with visibility through the camera (12, see FIG. 2) by moving dust or other debris 16 in front of the camera lens. Accordingly, it is advantageous to be able to vary the fluid flow field 52 during use of the ureteroscope 4.

FIG. 9B illustrates the same ureteroscope 4 and access sheath 4 as FIG. 9A, but the first irrigation fluid channel 7a is closed or turned off, and the second irrigation channel 7b is opened or turned on. As illustrated, this alters the fluid flow field 52 through the treatment site 5. Advantageously, this may help to dislodge kidney stones 15 or move dust/debris 16 away from the camera lens.

FIG. 9C illustrates the fluid flow field 52a, 52b when both the first and second irrigation fluid channels 7a, 7b are opened or turned on, providing a third fluid flow field 52a, 52b at the treatment site 5.

It will be appreciated that operating the first and/or second irrigation fluid channels 7a, 7b individually or together, or at different pressures or volumetric flow rates, as illustrated in FIGS. 9A to 9C, varies the fluid flow field 52 by changing the relative positions of the operational irrigation fluid channel(s) 7a, 7b and the suction channel 8 at the treatment site 5.

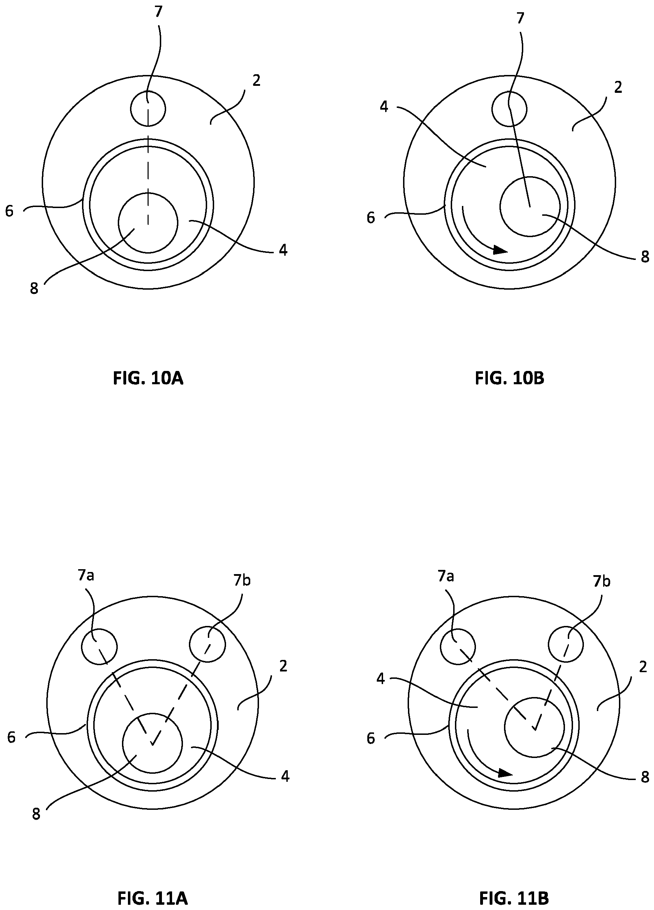

FIGS. 10A to 11B illustrate alternative example access sheaths 2 and ureteroscopes 4 that can be operated to vary the fluid flow field 52.

In the example of FIGS. 10A and 10B the access sheath 2 has a single irrigation fluid channel 7a, and the ureteroscope 4 has an eccentric suction channel 8 (i.e., the suction channel 8 is non-centrally disposed on the ureteroscope 4). Accordingly, as illustrated in FIGS. 10A and 10B, rotation of the ureteroscope 4 within the ureteroscope channel 6 of the access sheath 2 changes the relative positions of the irrigation fluid channel 7 and the suction channel 8, thereby varying the fluid flow field at the treatment site. FIG. 10A shows the ureteroscope 4 in a first rotational position, and FIG. 10B shows the ureteroscope in a second rotational position.

The example of FIGS. 11A and 11B are similar to the example of FIGS. 10A and 10B, but the access sheath 2 has first and second irrigation fluid channels 7a, 7b. Rotation of the ureteroscope 4 within the ureteroscope channel 6 of the access sheath 2 changes the relative positions of the first and second irrigation fluid channels 7a, 7b and the suction channel 8, thereby varying the fluid flow field at the treatment site. In this example, the first and second irrigation fluid channels 7a, 7b may be operated (i.e., turned on and off) independently, or at different pressures or volumetric flow rates, as described with reference to FIGS. 9A to 9C to provide additional possibilities to vary the fluid flow field at the treatment site.

It will be appreciated that rotation of the ureteroscope 4 within the ureteroscope channel 6 of the access sheath 2 will change the relative positions of the (operational) irrigation fluid channel(s) 7a, 7b and the suction channel 8 for any of the access sheaths 2 described herein, particularly those described with reference to FIGS. 2, 3A to 3E, and 4A to 4F.

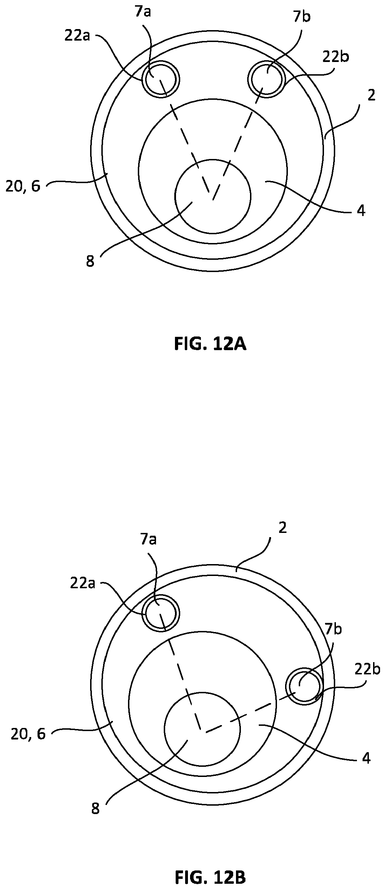

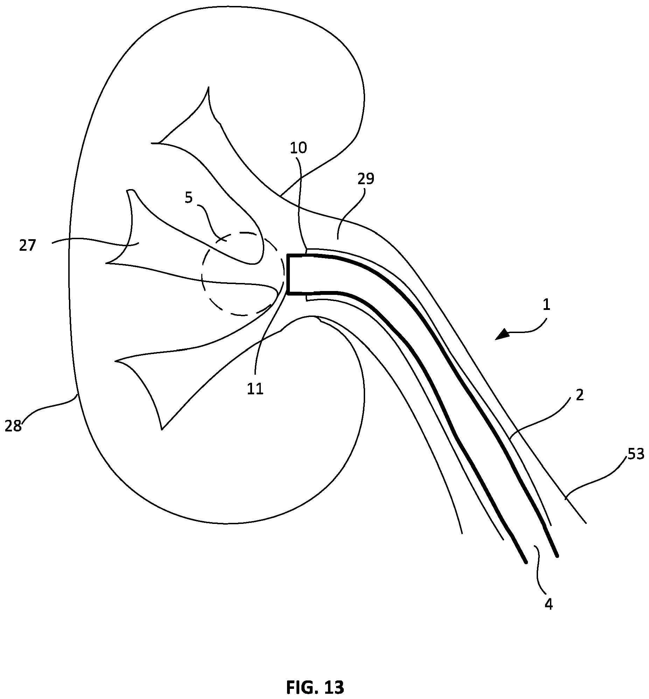

FIGS. 12A and 12B illustrate a further example of changing the relative positions of the (operational) irrigation fluid channel(s) 7a, 7b and the suction channel 8 for an access sheath 2 having separate tubes 22a, 22b for the irrigation fluid channels 7a, 7b. In this example, the ureteroscope 4 and the tubes 22a, 22b that form the irrigation fluid channels 7a, 7b are each independently movable within the lumen 20 of the access sheath 2 to change their relative positions at the distal end of the ureteroscope 4 and access sheath 2. The tube 22a, 22b and/or ureteroscope 4 positions may be altered by twisting them relative to each other at the proximal ends, for example at the adapter (35, see FIG. 7A). FIGS. 13 to 15B illustrate further examples of the apparatus for ureteroscopy 1. In these examples the access sheath 2 and ureteroscope 4 may be the access sheath 2 and ureteroscope 4 as described with reference to any of the other examples described herein.

As shown in FIG. 13, in some examples the distal end 10 of the access sheath 2 is positioned in the renal pelvis 29 or inside the kidney 28, particularly in a calyx 27, during use. Advantageously, this means that irrigation fluid is provided directly to the treatment site 5 where the ureteroscope 4 is working via the or each irrigation fluid channel (7, see FIG. 1A) in the access sheath 2. This can help to maintain visibility via the camera and to move/dislodge/clear objects and debris, as previously described, at the treatment site 5.

In known ureteroscope systems, an access sheath 2 is inserted only as far as the ureter 53, or at most partly into the renal pelvis 29, and the ureteroscope 4 is moves past the end of the access sheath 2 to the treatment site 5. In the some examples of the present disclosure, the distal end 10 of the access sheath 2 is positioned at the treatment site 5, or as near as possible, so that the irrigation fluid is provided at the treatment site 5 and the fluid flow field can be controlled accordingly.

As described hereinafter, the access sheath 2 may be coupled to the ureteroscope 4 at or near a distal end 10 of the access sheath 2 and the distal end 11 of the ureteroscope 4 during use. In particular, as the ureteroscope 4 is inserted into the access sheath 2 during use it couples to the access sheath 2 as the distal end 11 of the ureteroscope 4 reaches or approaches the distal end 10 of the access sheath 2. Thereafter, continuing to move the ureteroscope 4 towards the kidney will move the access sheath 2 with it to the desired position. As described below, the ureteroscope 4 may decouple from the access sheath 2 as it is moved back away from the kidney 28 to remove it from the patient after use. The access sheath 2 and/or the ureteroscope 4 has a coupling mechanism to provide this function.

The coupling mechanism may selectively decouple the access sheath 2 and the ureteroscope 4.

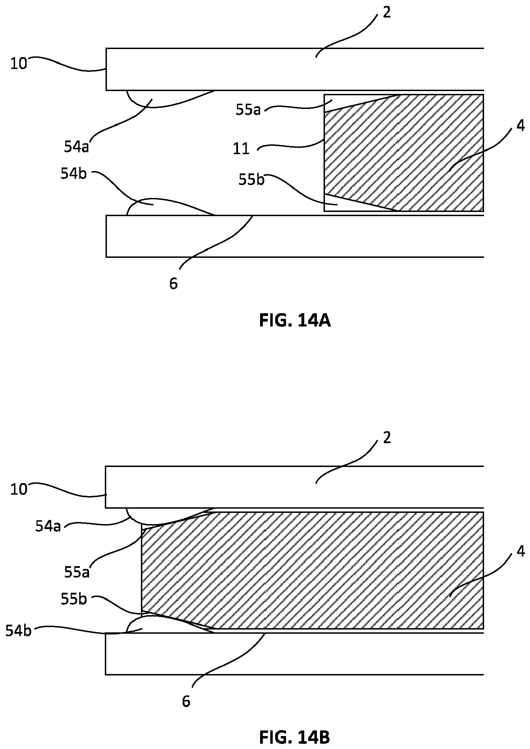

FIGS. 14A and 14B illustrate an example coupling mechanism. As illustrated, the access sheath 2 comprises one or more, in this example two, coupling members 54a, 54b extending from a sidewall of the ureteroscope channel 6 into the ureteroscope channel 6. The coupling members 54a, 54b are arranged to engage the ureteroscope 4 as the distal end 11 of the ureteroscope 4 reaches the coupling member(s) 54a, 54b during use. The coupling member(s) 54a, 54b are disposed at or near the distal end 10 of the access sheath 2.

Optionally, the ureteroscope 4 has recesses 55a, 55b corresponding to the coupling members 54a, 54b. The recesses 55a, 55b may be formed in a side surface of the ureteroscope 4 and extend to the distal end 11 of the ureteroscope 4, as illustrated.

In use, as the ureteroscope 4 is passed along the ureteroscope channel 6 of the access sheath 2 the ureteroscope 4 will engage the coupling members 54a, 54b of the access sheath 2. Further movement of the ureteroscope 4 towards kidney will carry the access sheath 2 along with it. Accordingly, the distal end 10 of the access sheath 2 (with the irrigation fluid channel(s) (7, see FIG. 1A) as previously described) maintains a position in line with, or adjacent to, the distal end 11 of the ureteroscope 4.

Optionally, the ureteroscope 4 includes an attachment at the distal end 11 for providing the recesses 55a, 55b. In examples where the irrigation fluid channel(s) are provided in a space between the ureteroscope 4 and the access sheath 2, the attachment may provide an outlet for the irrigation fluid channel(s) 7.

After the ureteroscopy is complete the ureteroscope 4 can be removed from the access sheath 2 and the coupling members 54a, 54b will disengage. Optionally, if the operator wanted to retract the position of the ureteroscope 4 and access sheath 2 in a direction away from the kidney together, for example to enter a different calyx, then the access sheath 2 and ureteroscope 4 can be pulled together at the proximal end (i.e., external of the patient). This can be achieved, for example, by moving the adapter (35, see FIG. 6A) and operation portion (4a, see FIG. 6A) of the ureteroscope 4.

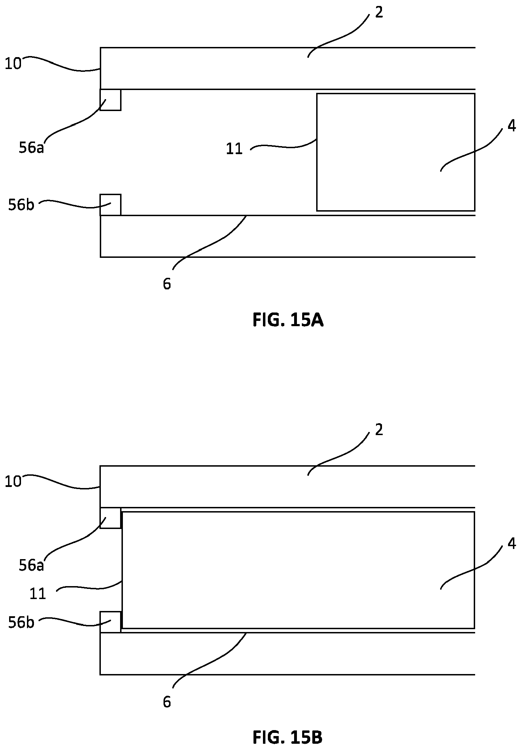

In the example of FIGS. 15A and 15B, the access sheath 2 comprises one or more, in this example two, coupling members 56a, 56b at a distal end 11 of the ureteroscope channel 6 of the access sheath 2. Alternatively, the access sheath 2 may comprise a coupling member 56 in the form of a circumferential (or part-circumferential) ring at the distal end 10 of the ureteroscope channel 6 that defines a restricted opening at the end of the access sheath 2.

In use, as the ureteroscope 4 is passed along the ureteroscope channel 6 of the access sheath 2 the ureteroscope 4 will abut the coupling member(s) 56a, 56b of the access sheath 2. Further movement of the ureteroscope 4 towards kidney will move the access sheath 2 along with it. Accordingly, the distal end 10 of the access sheath 2 (with the irrigation fluid channel(s) (7, see FIG. 1A) as previously described) maintains a position in line with, or adjacent to, the distal end 11 of the ureteroscope 4.

After the ureteroscopy is complete the ureteroscope 4 can be removed from the access sheath 2 and the distal end 11 of the ureteroscope 4 will move out of abutment with the coupling member(s) 56a, 56b. Optionally, if the operator wanted to retract the position of the ureteroscope 4 and access sheath 2 in a direction away from the kidney together, for example to enter a different calyx, then the access sheath 2 and ureteroscope 4 can be pulled together at the proximal end (i.e., external of the patient). This can be achieved, for example, by moving the adapter (35, see FIG. 6A) and operation portion (4a, see FIG. 6A) of the ureteroscope 4.

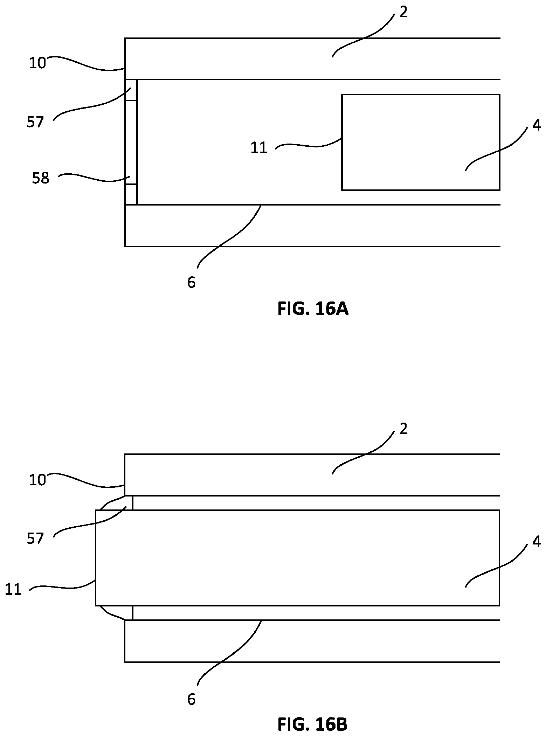

FIGS. 16A and 16B illustrate a different example coupling member 57 for coupling the ureteroscope 4 to the access sheath 2 at or near a distal ends 10, 11 of the access sheath 2 and ureteroscope 4. In this example, the access sheath 2 comprises a resiliently deformable annular membrane 57 disposed at or near the distal end 10 of the ureteroscope channel 6. The resiliently deformable annular membrane 57 has an opening 58 that is configured to deform about the distal end 11 of the ureteroscope 4 to grip the ureteroscope 4 and couple the ureteroscope 4 to the access sheath 2.

In use, as the ureteroscope 4 is passed along the ureteroscope channel 6 of the access sheath 2 the ureteroscope 4 will deform the resiliently deformable annular membrane 57 of the access sheath 2 as it passes at least partly through the opening 58. Further movement of the ureteroscope 4 towards kidney will carry the access sheath 2 along with it. Accordingly, the distal end 10 of the access sheath 2 (with the irrigation fluid channel(s) (7, see FIG. 1A) as previously described) maintains a position in line with, or adjacent to, the distal end 11 of the ureteroscope 4.

After the ureteroscopy is complete the ureteroscope 4 can be removed from the access sheath 2 by pulling the ureteroscope 4 while holding the access sheath 2 (or adapter 35, see FIG. 6A) in place. The ureteroscope 4 will move out of engagement with the annular membrane 57 and can be removed. Optionally, if the operator wanted to retract the position of the ureteroscope 4 and access sheath 2 in a direction away from the kidney together, for example to enter a different calyx, then the access sheath 2 and ureteroscope 4 can be moved together by pulling either the ureteroscope 4 or the access sheath 2 (or adapter 35, see FIG. 6A) at the proximal end (i.e., external of the patient). The grip provided by the resiliently deformable annular membrane 57 provides coupling in both directions until it is decoupled by moving the ureteroscope 4 relative to the access sheath 2. Advantageously, this additionally provides the option for controlling the proportion of the ureteroscope 4 that extends past the distal end 10 of the access sheath 2, which can be increased by holding the access sheath 2 (or the adapter 35, see FIG. 6A) at the proximal end and pushing the ureteroscope 4 inwards where it moves further through the opening 58 of the resiliently deformable annular membrane 57.

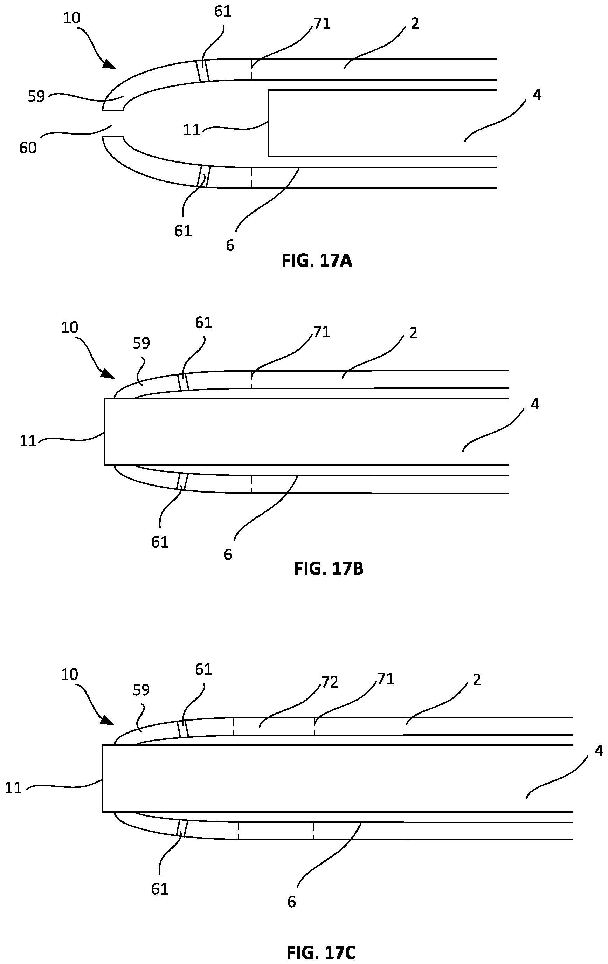

As shown in FIG. 17A and FIG. 17B, a distal end 10 of the access sheath 2 may comprise a resiliently deformable tapered end 59 having an opening 60 to receive the distal end 11 of the ureteroscope 4 as the ureteroscope 4 is pushed through the ureteroscope channel 6 during use. As illustrated, the resiliently deformable tapered end 59 deforms about the distal end 11 of the ureteroscope 4 and gips thereon.

Once coupled, similar to the example of FIGS. 16A and 16B, further movement of the ureteroscope 4 towards kidney will move the access sheath 2 along with it. Accordingly, the distal end 10 of the access sheath 2 (with the irrigation fluid channel(s) (7, see FIG. 1A) as previously described) maintains a position in line with, or adjacent to, the distal end 11 of the ureteroscope 4.

After the ureteroscopy is complete the ureteroscope 4 can be removed from the access sheath 2 by pulling the ureteroscope 4 while holding the access sheath 2 (or adapter 35, see FIG. 6A) in place. The ureteroscope 4 will move out of engagement with the resiliently deformable tapered end 59 and can be removed. Optionally, if the operator wanted to retract the position of the ureteroscope 4 and access sheath 2 in a direction away from the kidney together, for example to enter a different calyx, then the access sheath 2 and ureteroscope 4 can be moved together by pulling either the ureteroscope 4 or the access sheath 2 (or the adapter 35, see FIG. 6A) at the proximal end (i.e., external of the patient). The grip provided by the resiliently deformable tapered end 59 provides coupling in both directions until it is decoupled by moving the ureteroscope 4 relative to the access sheath 2. Advantageously, this additionally provides the option for controlling the proportion of the ureteroscope 4 that extends past the distal end 10 of the access sheath 2, which can be increased by holding the access sheath 2 (or adapter 35, see FIG. 6A) at the proximal end and pushing the ureteroscope 4 inwards where it moves through the opening 60 of the resiliently deformable tapered end 59.

In examples where the irrigation fluid channel(s) 7 is/are formed in the space between the ureteroscope 4 and the access sheath 2 (e.g., as shown in FIGS. 3A to 3F and FIGS. 4A to 4C, the access sheath 2 may comprise one or more irrigation fluid outlets 61, as illustrated in FIGS. 17A and 17B for the irrigation fluid.

In examples where the irrigation fluid channel(s) 7 is formed as separate lumens in the access sheath 2 (e.g., as shown in FIGS. 1B, 4C, and 4D) the access sheath 2 can comprise a similar irrigation fluid outlet 61 at or near the distal end 10 of the access sheath 2 that connects to the or each lumen.

In a further example, as illustrated in FIGS. 17A to 17C, a distal end portion 71 of the access sheath 2 is resiliently deformable such that after the ureteroscope 4 has coupled to the access sheath 2 it can be flexed and optionally extended relative to the remainder of the access sheath 2. Flexing of the distal end portion 71 of the access sheath 2 allows for articulation of the ureteroscope 4 during use. The access sheath 2 may be made from a resiliently deformable material with a stiffening member, for example a coiled metal wire, running through the resiliently deformable material to increase the stiffness of the access sheath 2. The stiffening member may have a lower stiffness in the distal end portion 71 of the access sheath 2, for example the coiled metal wire may have fewer turns by unit length in the distal end portion 71 to provide reduced stiffness and increased flexibility.

Extension of the distal end portion 71 from the remainder of the access sheath 2 allows for the distal end 10 of the access sheath 2 to be moved, with the distal end 11 of the ureteroscope 4, further into the kidney and the treatment site 5 such that irrigation fluid is provided at the treatment site 5. FIG. 17C shows the distal end portion 71 of the access sheath 10 in the extended state.

In the example of FIGS. 17A to 17C the ureteroscope 4 may comprise an attachment at the distal end 11 of the ureteroscope 4 that couples to the resiliently deformable tapered end 59 during use.

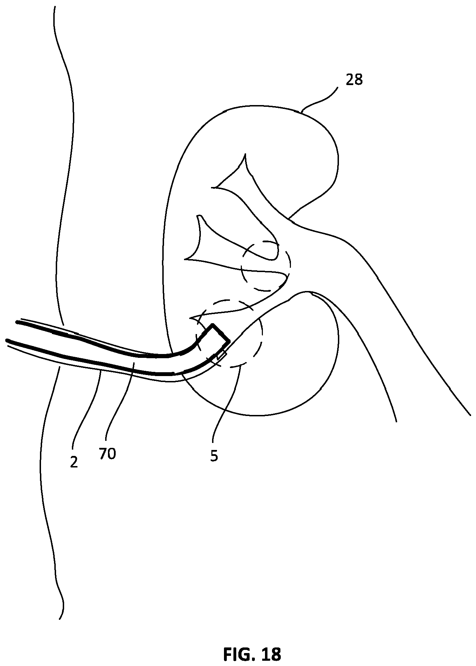

The examples described above relate to apparatus for ureteroscopy, but it will be appreciated that the access sheath 2 may be used with other surgical tools in other endourological procedures, for example during percutaneous nephrolithotomy (PCNL), as described with reference to FIG. 18.

FIG. 18 illustrates a percutaneous nephrolithotomy (PCNL) procedure, and use of the access sheath 2 in such a procedure. In this PCNL procedure, an opening is formed in a patient's body providing access to the kidney 28. This is in contrast to ureteroscopy which uses the urethra, bladder, and ureter to access the kidney. During a PCNL procedure an opening is typically formed in a patient's back, and an access sheath 2 is inserted through the opening and into the kidney 28 to provide access for a surgical tool, such as an endoscope, basket, etc....

The access sheath 2 used in PCNL can be substantially the same as the example access sheaths 2 described with reference to the other examples described herein. An access sheath for use in a PCNL procedure may have different dimensions than an access sheath for ureteroscopy, but otherwise they are similar or the same.

Surgical tools 70, such as an endoscope or basket, can be passed through the access sheath 2 to access a treatment site 5 in the kidney 28. As per the other examples, irrigation fluid can be provided through one or more irrigation fluid channels in the access sheath 2, and the surgical tool 70 has a suction channel for extraction of fluid and debris from the treatment site 5, in particular the kidney 28. This provides the same advantages as set out for ureteroscopy in the examples of FIGS. 1A to 16.

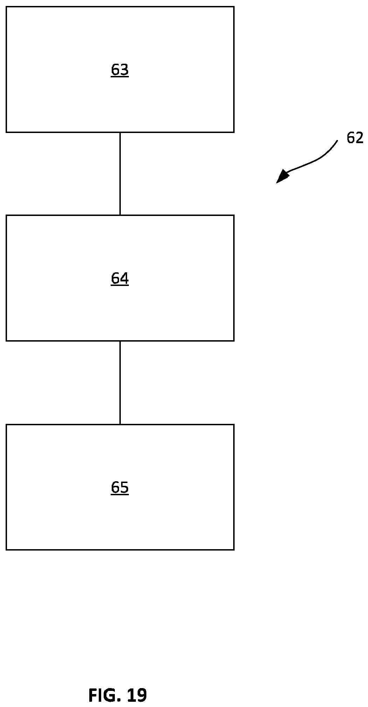

FIG. 19 illustrates a endourological procedure 62, for example a ureteroscopy or PCNL procedure. The method comprises positioning an access sheath in a patient's urinary system 63. The access sheath may be any access sheaths 2 described with reference to the examples, and includes at least one irrigation fluid channel 7. As explained previously, during some ureteroscopy procedures the access sheath is positioned so that a distal end of the access sheath is positioned in the renal pelvis, or inside the kidney, in particular in a calyx. During a PCNL procedure the access sheath passes through a surgical opening in the body and into the kidney.

The method further comprises passing a surgical tool through the access channel of the access sheath to the treatment site 64. As described previously, the surgical tool has a suction channel. In ureteroscopy the surgical tool may be a ureteroscope, and in PCNL the surgical tool may be an endoscope.

The method also includes conveying irrigation fluid to the treatment site through the one or more irrigation fluid channels in the access sheath, and extracting fluid (and debris) from the treatment site through the suction channel of the surgical tool 65.

In examples, the method of ureteroscopy 62 may be a method of lithotripsy and may further comprise laser ablating an object at the treatment site, for example a kidney stone.

As explained above, the method 62 may include controlling conveyance of the irrigation fluid to the treatment site to vary a fluid flow field at the treatment site. In particular, the method may include selectively controlling (e.g., opening, closing, or varying a fluid pressure or volumetric flow rate through) each of a plurality of irrigation fluid channels to vary the fluid flow field at the treatment site.

As explained above, the method 62 may include rotating the surgical tool, in particular the ureteroscope, within the access sheath to vary the relative positions of the suction channel and the or each irrigation fluid channel at the treatment site, to vary a fluid flow field at the treatment site.

As previously explained, the method 62 may include controlling an irrigation fluid system and a suction system in dependence of each other to balance the flow of irrigation fluid towards the treatment site via the or each irrigation channel, and the flow of fluid away from the treatment site via the suction channel.

Controlling the irrigation fluid system and suction system may include receiving an input from an operator to change a flow rate of the irrigation fluid through the or each irrigation fluid channel, and adjusting the flow rate of the irrigation fluid and the suction pressure generated in the suction channel in response to the input.

Controlling the irrigation fluid system and suction system may include receiving an input from an operator to change a suction pressure of the suction channel, and adjusting the suction pressure and the flow rate of the irrigation fluid in response to the input.

Conveying irrigation fluid to the treatment site through the one or more irrigation fluid channels in the access sheath and extracting fluid (and debris) from the treatment site through the suction channel of the surgical tool 65 may comprise controlling one or both of the irrigation fluid flow rate and the suction pressure based on a sensor signal received from one or more sensors arranged to detect fluid characteristic within the apparatus and/or the treatment site.

As described with reference to FIG. 8, the apparatus 1 may include one or more sensors 46, 47, 48, 49, 51 for detecting a fluid characteristic within the apparatus 1 and/or the treatment site 5. The fluid characteristic may be a fluid temperature or fluid pressure at the treatment site (i.e., at the distal end of the ureteroscope) or in the irrigation fluid system or suction system. Additionally or alternatively, the fluid characteristic may be a volumetric fluid flow rate in the irrigation fluid system or in the suction system.

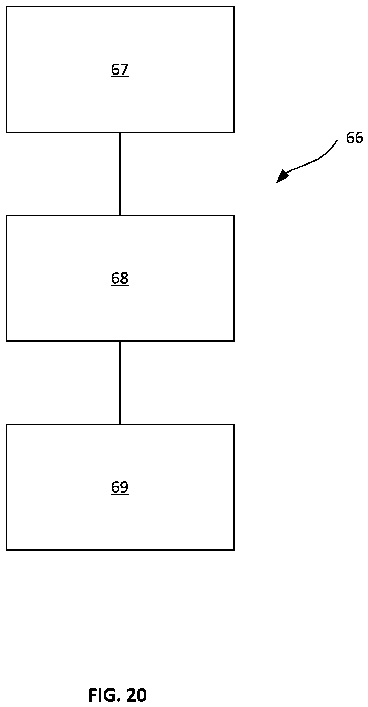

FIG. 20 illustrates a method of ureteroscopy 66, in particular lithotripsy. The method 66 includes positioning an access sheath in a patient's urinary system and then passing a surgical tool, in particular a ureteroscope, through an access channel of the access sheath 67. The access sheath may be any of the access sheaths described herein.

The access sheath and/or surgical tool comprises a coupling mechanism, and the method includes coupling the surgical tool to the access sheath as the surgical tool is passed along the access sheath 68.

Thereafter, the method comprises moving the surgical tool and access sheath together towards the kidney 69.

In examples, the surgical tool and access sheath are moved together until the distal end of the access sheath is positioned in the renal pelvis or inside the kidney, in particular in a calyx.

The method 66 may further comprise decoupling the surgical tool from the access sheath and removing the surgical tool from the access sheath.

As described above, the surgical tool has a suction channel and the access sheath has one or more irrigation fluid channels. The method 66 includes conveying irrigation fluid to the treatment site via the one or more irrigation fluid channels, and extracting fluid from the treatment site via the suction channel, as per the method of FIG. 19. The method of FIG. 20 may further include any additional or optional aspect described with reference to FIG. 19.

The methods 62, 66 of FIG. 19 or FIG. 20 may further include varying or controlling a fluid flow field at the treatment site, for example by varying the relative positions of an operational irrigation fluid channel and a suction channel. This may be achieved through any of the apparatus described herein. Varying or controlling the fluid flow field at the treatment site may include changing a volumetric flow rate of the irrigation fluid through the irrigation fluid channels and/or changing a suction pressure in the suction channel.

Throughout the description and claims of this specification, the words "comprise" and "contain" and variations of them mean "including but not limited to", and they are not intended to (and do not) exclude other components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics or groups described in conjunction with a particular aspect, embodiment or example of the disclosure are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The disclosure is not restricted to the details of any foregoing embodiments. The disclosure extends to any novel one, or any novel combination, of the features disclosed in this specification (including any accompanying claims, abstract and drawings), or to any novel one, or any novel combination, of the steps of any method or process so disclosed.

## Claims

1. An access sheath for use in an endourological procedure, wherein the access sheath is configured for use with a surgical tool having a suction channel for extraction of fluid from a treatment site during use, the access sheath being positionable within a patient's urinary system during use to provide access to the treatment site for the surgical tool,
wherein the access sheath comprises:
an access channel extending to a distal end of the access sheath, the access channel being configured to receive the surgical tool such that a distal end of the surgical tool passes through the access channel and is positioned at the treatment site during use, and
an irrigation fluid channel configured to convey irrigation fluid to the treatment site during use such that irrigation fluid is provided to the treatment site via the irrigation fluid channel of the access sheath and is extracted via the suction channel of the surgical tool.

2. The access sheath of claim 1, comprising a plurality of irrigation fluid channels, each of the plurality of irrigation fluid channels being configured to convey irrigation fluid to the treatment site during use.

3. The access sheath of any preceding claim, wherein the access channel and the or each irrigation fluid channel are movable relative to each other within the access sheath such that the respective positions of the access channel and the or each irrigation channel can be altered to vary a fluid flow characteristic at the treatment site.

4. The access sheath of any preceding claim, wherein the access channel and the or each irrigation fluid channel are separate lumens in the access sheath.

5. The access sheath of any of claims 1 to 3, wherein the access sheath comprises a lumen extending to the distal end of the access sheath, the lumen having a non-circular cross-section configured to constrain the position of the surgical tool during use, and wherein the or each irrigation fluid channel is defined within the lumen in an area surrounding the surgical tool during use.

6. The access sheath of any preceding claim, further comprising a coupling mechanism configured to couple the access sheath to the surgical tool during use, the coupling mechanism being disposed at or near the distal end of the access sheath.

7. An access sheath adapter for use with the access sheath of any of claims 1 to 6 during an endourological procedure, the access sheath adapter comprising a connection portion for connecting to a proximal end of the access sheath external of the patient during use, an opening providing access for the surgical tool, and an irrigation fluid supply connector for connecting the or each irrigation fluid channel to a source of irrigation fluid.

8. The access sheath adapter of claim 7, wherein the access sheath comprises a plurality of irrigation fluid channels, and wherein the irrigation fluid supply connector comprises independent fluid connections between the source of irrigation fluid and each of the plurality of irrigation fluid channels.

9. Apparatus for use in an endourological procedure, the apparatus comprising:
a surgical tool comprising a suction channel for extraction of fluid from a treatment site during use, and
an access sheath positionable within a patient's urinary system during use to provide access to the treatment site for the surgical tool, the access sheath comprising:
an access channel extending to a distal end of the access sheath, the access channel being configured to receive the surgical tool such that a distal end of the surgical tool passes through the access channel and is positioned at the treatment site during use, and
an irrigation fluid channel configured to convey irrigation fluid to the treatment site during use such that irrigation fluid is provided to the treatment site via the irrigation fluid channel of the access sheath and is extracted via the suction channel of the surgical tool.

10. The apparatus of claim 9, further comprising an irrigation fluid system configured to supply irrigation fluid to the or each irrigation fluid channel.

11. The apparatus of claim 10, wherein the access sheath comprises a plurality of irrigation fluid channels, and the irrigation fluid system is configured to control supply of irrigation fluid to each of the plurality of irrigation fluid channels independently of each other.

12. The apparatus of claim 10 or claim 11, further comprising:
a suction system arranged to generate suction in the suction channel of the surgical tool, and
a fluid control system configured to control the irrigation fluid system and the suction system.

13. The apparatus of claim 12, wherein the fluid control system is configured to balance the flow rate of fluid towards, and away from, the treatment site.

14. The apparatus of any of claims 10 to 12, further comprising an access sheath adapter configured to be connected to a proximal end of the access sheath external of the patient during use, and wherein the access sheath adapter comprises an opening providing access for the surgical tool, and an irrigation fluid supply connector for connecting the or each irrigation fluid channel to the irrigation fluid system.

15. The apparatus of claim 14, wherein the access sheath comprises a plurality of irrigation fluid channels, and wherein the irrigation fluid supply connector comprises independent fluid connections between the irrigation fluid system and each of the plurality of irrigation fluid channels.
